# EUROPEAN PATENT SPECIFICATION

(11) **EP 4 324 416 B1**
(45) Date of publication and mention of the grant of the patent: **18.11.2026**
(21) Application number: 22807873.9
(22) Date of filing: 13.05.2022
(51) Int. Cl.: A61B 18/12, A61B 18/14, A61N 1/06, A61N 1/32, A61N 1/20, A61B 18/00, A61N 1/40, A61N 1/04

(54) **HIGH FREQUENCY CURRENT OUTPUT DEVICE ENABLING SEQUENTIAL OUTPUT AND CONTINUOUS OUTPUT OF MONOPOLAR TYPE CURRENT AND BIPOLAR TYPE CURRENT**
HOCHFREQUENZSTROMAUSGABEVORRICHTUNG MIT SEQUENTIELLEM AUSGANG UND KONTINUIERLICHEM AUSGANG VON MONOPOLAREM STROM UND BIPOLAREM STROM
DISPOSITIF DE SORTIE DE COURANT HAUTE FRÉQUENCE PERMETTANT UNE SORTIE SÉQUENTIELLE ET UNE SORTIE CONTINUE DE COURANT DE TYPE MONOPOLAIRE ET DE COURANT DE TYPE BIPOLAIRE

(30) Priority: 14.05.2021 KR 20210062598; 12.05.2022 KR 20220058277
(43) Date of publication of application: 21.02.2024
(73) Proprietor: Jeisys Medical Inc., Geumcheon-gu Seoul 08501 (KR)
(72) Inventor: JANG, Miran, Seoul 08501 (KR); SUN, Hye Jin, Seoul 08501 (KR); AN, Kyoungho, Seoul 08501 (KR); KANG, Dong Hwan, Seoul 08501 (KR)
(74) Representative: dompatent
(86) International application number: PCT/KR2022/006866
(87) International publication number: WO 2022/240226

(56) References cited:
- WO-A1-2020/086552
- JP-A- 2008 253 782
- JP-A- 2009 512 496
- KR-A- 20130 010 965
- KR-A- 20130 106 020
- US-A- 4 171 700
- US-A- 4 171 700
- US-A1- 2007 088 413
- US-A1- 2018 000 533

## Description

### [TECHNICAL FIELD]

Embodiments of the inventive concept described herein relate to a high frequency current output device, and more particularly, relate to a device that increases collagen or elastin by sequentially or continuously delivering a monopolar-type & bipolar-type pattern or a bipolar type & monopolar-type pattern at one time when a high frequency current is delivered to skin.

### [BACKGROUND ART]

At present, a high frequency current output device is being used to perform high frequency irradiation for tightening, acne, and pigmentation .

For example, the high frequency current output device may be operated in an invasive method of delivering a high frequency current to skin after a hole is made in the skin by using a needle, and a non-invasive method of delivering a high frequency current to a skin surface.

Generally, a method of delivering high frequency energy may have a monopolar type and a bipolar type. One operating device selectively uses a monopolar-type method or a bipolar-type method depending on high frequency irradiation depth of skin to which the high frequency energy is irradiated.

In this way, the mechanism of high frequency irradiation using a high frequency current may be described in two ways.

First, heating of dermal collagen fibers causes contraction of the skin and entire subcutaneous tissue. Second, collagen coagulation denaturation promotes collagen regeneration.

The bipolar-type method has limited heating amount and penetration depth. On the other hand, the monopolar-type method may use a sufficient amount of energy.

The bipolar-type method is well known to be effective for freckles, capillary dilatation, pore enlargement, mild wrinkles, and the monopolar-type method is well known to be effective for wrinkles and sagging skin.

As such, because the depth at which high frequency energy is delivered in the bipolar-type method is different from the depth at which high frequency energy is delivered in the monopolar-type method, the bipolar-type method and the monopolar-type method are selected and used depending on the purpose of high frequency irradiation .

However, when a pattern having the combination of two or more types is selected, an additional manipulation is required to change from the monopolar-type to the bipolar-type or from the bipolar-type to the monopolar-type. A device failed to be supplied to output monopolar-type and bipolar-type currents or bipolar-type and monopolar-type currents simultaneously or with a short relay time in one operation.

Moreover, one high frequency current output device may select and use the monopolar-type or the bipolar-type. However, in a case of the monopolar-type, when all needles are provided with the same polarity, an electrode deflection phenomenon may occur due to the proximity effect of needles.

Accordingly, there may be required for devices and research capable of maximizing effects of high frequency irradiation such that monopolar-type and bipolar-type currents or bipolar-type and monopolar-type currents are continuously output in one pattern.

A similar high frequency output device for delivering a high frequency current to skin is known from US20070088413A1, disclosing an apparatus and methods for delivering energy at multiple selectable tissue depths as selected by a clinician. The treatment apparatus includes at least two electrodes that are electrically isolated from each other, which permits each electrode to be independently energized for selecting different treatment depths. The electrodes may be concurrently energized with high frequency energy of the same polarity in a monopolar mode to deliver energy at a relatively deep depth into a patient's tissue, or with high frequency energy of a different polarity in a bipolar mode to provide a shallower penetration depth. US2018/000533A1 discloses a high frequency output device according to the pre-amble of claim 1.

### [DETAILED DESCRIPTION OF THE INVENTION]

### [TECHINICAL PROBLEM]

Embodiments of the inventive concept provide a high frequency current output device capable of continuously outputting a monopolar-type and bipolar-type of high frequency currents or a bipolar-type and monopolar-type of high frequency currents in one operation of the device.

Embodiments of the inventive concept provide a high frequency current output device capable of being applied to both invasive electrodes and non-invasive electrodes to be modified depending on a user's purpose.

Problems to be solved by the inventive concept are not limited to the problems mentioned above, and other problems not mentioned will be clearly understood by those skilled in the art from the following description.

### [TECHNICAL SOLUTION]

According to the invention a high frequency output device according to claim 1 is provided. Preferred embodiments are defined in the dependent claims. The methods disclosed herein do not form part of the claimed invention.

According to an embodiment, a high frequency output device includes an electrode unit including one or more first electrodes and one or more second electrodes, a first high frequency power generation unit, a switching circuit, and a control unit that controls a switching operation of the switching circuit so as to output, based on power generated from the first high frequency power generation unit, a monopolar-type current, in which the same polarity is applied to the first electrodes and the second electrodes, or a bipolar-type current, in which currents of different polarities are applied.

According to an embodiment a high frequency output device includes an electrode unit including a first electrode group composed of the one or more first electrodes and a second electrode group composed of the one or more second electrodes. The control unit that allows operations of the first high frequency power generation unit and the second high frequency power generation unit, which is connected to a separate electrode from the first high frequency power generation unit to supply a current, to be turned on or off such that the monopolar-type current and the bipolar-type current are applied to the electrode unit simultaneously or sequentially. The first electrode group is connected to the first high frequency power generation unit to form a closed circuit in correspondence with the NE pad for an output of the monopolar-type current. The second electrode group is connected to the second high frequency power generation unit, and is an electrode for an output of the bipolar-type current.

Other details according to an embodiment of the inventive concept are included in the detailed description and drawings.

### [ ADVANTAGEOUS EFFECTS OF THE INVENTION]

According to an embodiment of the inventive concept, a high frequency current output device is capable of continuously outputting a monopolar-type and bipolar-type of high frequency currents or a bipolar-type and monopolar-type of high frequency currents in one operation of the device.

Moreover, according to an embodiment of the inventive concept, a high frequency current output device is capable of being applied to both invasive electrodes and non-invasive electrodes to be modified and used depending on a user's purpose.

Furthermore, according to an embodiment of the inventive concept, a high frequency current output device is capable of preparing for a leakage current between electrodes by placing a plurality of electrodes in a non-overlapped shape when the plurality of electrodes are alternately placed, as the plurality of electrodes within an electrode unit are formed to be of a thin film type.

Effects of the inventive concept are not limited to the effects mentioned above, and other effects not mentioned will be clearly understood by those skilled in the art from the following description.

### [ DESCRIPTION OF THE DRAWINGS]

FIG. 1 is a block diagram of a system including a high frequency current output device, according to an embodiment of the inventive concept.
FIG. 2 is an internal block diagram of a switching circuit within the skin treatment device shown in FIG. 1.
FIG. 3A is a vertical cross-sectional view of a plurality of electrodes, according to an embodiment of the inventive concept. FIG. 3B is a top plan view of a plurality of electrodes, according to an embodiment.
FIG. 4A is a vertical cross-sectional view when a plurality of electrodes are provided as non-invasive electrodes, according to an embodiment of the inventive concept. FIG. 4B is a top plan view of a plurality of electrodes in the case of FIG. 4A.
FIG. 5A is a top plan view of a plurality of electrodes provided as invasive electrodes, according to an embodiment. FIG. 5B is a side view of a plurality of electrodes in the case of FIG. 5A.
FIG. 6 is an image illustrating results of collagen production according to a monopolar-type current output or a bipolar-type current output, according to an embodiment of the inventive concept.
FIGS. 7 and 8 are diagrams showing a user interface provided by a high frequency current output device to control current outputs of a plurality of electrodes, according to an embodiment of the inventive concept.
FIG. 9 is a diagram showing a frequency heat transfer range when an electrode operates in a bipolar-type, operates in a monopolar-type, and operates in a monopolar-bipolar (MB) pattern, according to an embodiment of the inventive concept.
FIG. 10 is a block diagram for describing a case where a power output mode in the system shown in FIG. 1 corresponds to a bipolar-type.
FIG. 11 is a block diagram for describing a case where a power output mode in the system shown in FIG. 1 corresponds to a monopolar-type.
FIG. 12 is a block diagram illustrating a case where a power output mode in the system shown in FIG. 1 corresponds to a monopolar-type and a second high frequency power generation unit is additionally operated when a first high frequency power generation unit is operated.
FIG. 13 is a block diagram for describing a case where a power output mode in the system shown in FIG. 1 continuously outputs a monopolar-type current and a bipolar-type current.
FIG. 14 is a diagram obtained by comparing results of expression levels of factors related to skin aging when an electrode unit operates with respect to young skin and aged skin in the mono-mono pattern MM, the mono-bi pattern MB, the bi-mono pattern BM, and the bi-bi pattern BB, according to an embodiment of the inventive concept.
FIG. 15 is a block diagram of a system including a high frequency current output device, according to another embodiment of the inventive concept.
FIG. 16 is a block diagram for describing a case where a power output mode in the system shown in FIG. 15 corresponds to a bipolar-type.
FIG. 17 is a block diagram for describing a case where a power output mode in the system shown in FIG. 15 corresponds to a monopolar-type.
FIG. 18 is a conceptual diagram for describing a first embodiment of an impedance matching operation when a power output mode corresponds to the mono-bi pattern MB in the system shown in FIGS. 1 and 15.
FIG. 19 is a conceptual diagram for describing a second embodiment of an impedance matching operation when a power output mode corresponds to the mono-bi pattern MB in the system shown in FIGS. 1 and 15.
FIG. 20 is a block diagram of a system including a high frequency current output device, according to still another embodiment of the inventive concept.

### [ BEST MODE]

The above and other aspects, features and advantages of the inventive concept will become apparent from embodiments to be described in detail in conjunction with the accompanying drawings. The inventive concept, however, may be embodied in various different forms, and should not be construed as being limited only to the illustrated embodiments. Rather, these embodiments are provided as examples so that the inventive concept will be thorough and complete, and will fully convey the scope of the inventive concept to those skilled in the art. The inventive concept may be defined by the scope of the claims.

The terms used herein are provided to describe embodiments, not intended to limit the inventive concept. In the specification, the singular forms include plural forms unless particularly mentioned. The terms "comprises" and/or "comprising" used herein do not exclude the presence or addition of one or more other components, in addition to the aforementioned components. The same reference numerals denote the same components throughout the specification. As used herein, the term "and/or" includes each of the associated components and all combinations of one or more of the associated components. It will be understood that, although the terms "first", "second", etc., may be used herein to describe various components, these components should not be limited by these terms. These terms are only used to distinguish one component from another component. Thus, a first component that is discussed below could be termed a second component without departing from the technical idea of the inventive concept.

An NE pad used in this specification is a separate pad (NE pad) from a first electrode 310-N and a second electrode 320-N provided in the electrode unit 300, and refers to a neutral electrode pad.

An output setting pattern used in this specification refers to a pattern of an output current set by a combination of high frequency irradiation characteristics as a bipolar-type current and a monopolar-type current is applied simultaneously or sequentially.

A current output option used in this specification may be selected by a user through a user interface of a device, and the monopolar-type and/or bipolar-type output time or energy output intensity may be adjusted depending on the selected option. Unless otherwise defined, all terms (including technical and scientific terms) used herein have the same meaning as commonly understood by those skilled in the art to which the inventive concept pertains. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein.

Hereinafter, embodiments of the inventive concept will be described in detail with reference to accompanying drawings.

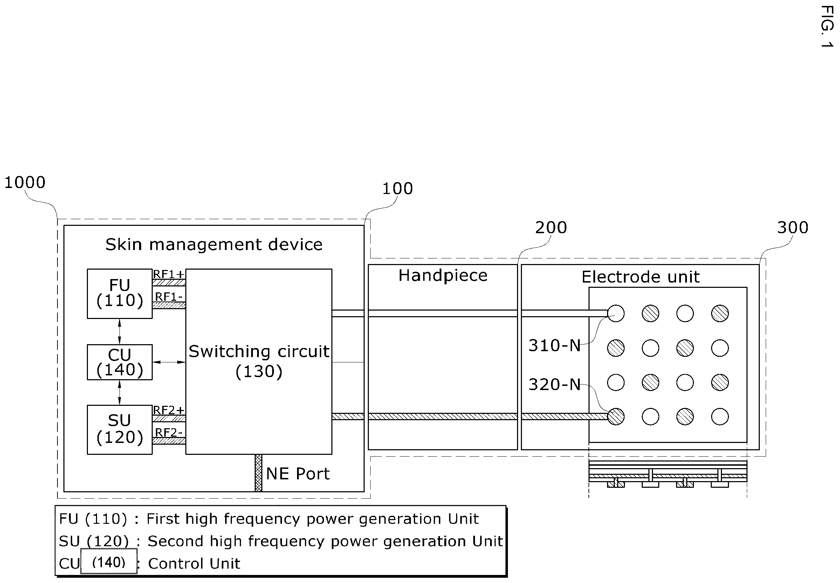

FIG. 1 is a block diagram of a system including a high frequency current output device 1000, according to an embodiment of the inventive concept, and the system includes a skin treatment device 100, a handpiece 200, an electrode unit 300, and the high frequency current output device 1000.

In this case, components of the high frequency current output device 1000 described above may be integrated into one main body, or at least some of the components may be separated from each other, and the separated components may exchange signals through wired or wireless communication.

The skin treatment device 100 includes a first high frequency power generation unit 110, a second high frequency power generation unit 120, a switching circuit 130, and a control unit 140.

In this case, in the skin treatment device 100, only the first high frequency power generation unit 110 is basically operated, and the second high frequency power generation unit 120 is additionally operated to prevent interference effects between a plurality of electrodes in close proximity within the electrode unit 300.

Here, the second high frequency power generation unit 120 is connected to a separate electrode from the first high frequency power generation unit 110 to supply a current.

Moreover, the electrode unit 300 includes a plurality of electrodes (i.e., a plurality of first electrodes 310-1 to 310-N and a plurality of second electrodes 320-1 to 320-N) electrically connected to a needle tip.

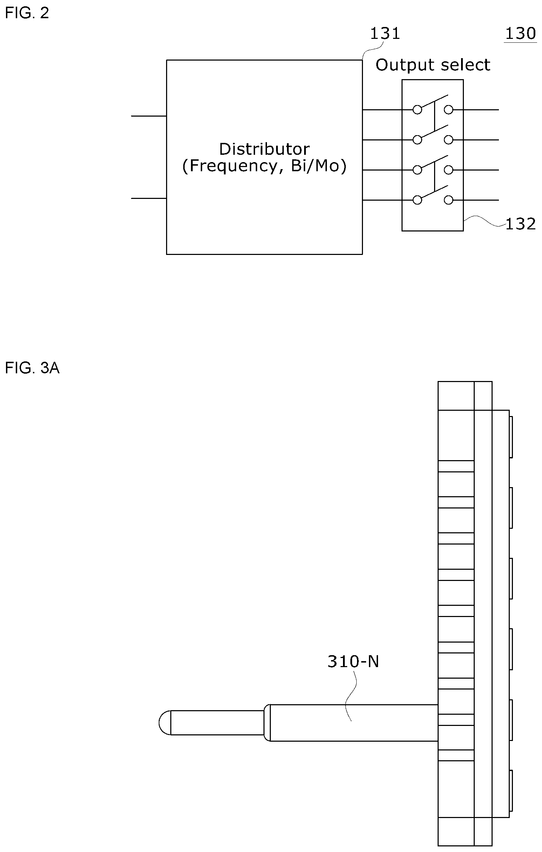

FIG. 2 is an internal block diagram of the switching circuit 130 in the skin treatment device 100 shown in FIG. 1, and the switching circuit 130 includes a distributor 131 and a relay 132.

The control unit 140 may be implemented with a memory, which stores data regarding an algorithm for controlling operations of the first high frequency power generation unit 110, the second high frequency power generation unit 120, and the switching circuit 130 in the skin treatment device 100 according to an embodiment of the inventive concept, or a program implementing the algorithm, and at least one processor (not shown) that performs operations described later by using data stored in the memory.

Here, the first high frequency power generation unit 110 and the second high frequency power generation unit 120 are only shown as being separated from each other to show an equal output between electrodes.

That is, the basic switching operation of the switching circuit 130 according to an embodiment of the inventive concept refers to switching an electrode connection between an RF electrode and an NE pad during high frequency irradiation, only through either the first high frequency power generation unit 110 and the second high frequency power generation unit 120.

In detail, when the first electrode 310-N and the second electrode 320-N are in the electrode unit 300, the first electrode 310-N and the second electrode 320-N are connected to the same polarity (e.g., (+)) in the case of a monopolar-type. The opposite polarity (e.g., (-)) is connected to the NE pad, and then the second electrode 320-N is connected to the opposite polarity and the NE pad is disconnected when the monopolar-type is switched to a bipolar-type.

Furthermore, even though the first electrode 310-N and the second electrode 320-N are connected to the same polarity as a monopolar-type output is provided, a section in which power is supplied to the first electrode 310-N and the second electrode 320-N is varied through the rapid on/off switching operation of the switching circuit 130.

Accordingly, in the monopolar-type, energy is output such that an interval between electrodes is narrow, and thus a skin phenomenon that energy is concentrated to the outside may be reduced.

According to this operating principle, in an embodiment of the inventive concept, the second high frequency power generation unit 120 is further provided in addition to the first high frequency power generation unit 110 to reduce the delay in switching to the first electrode 310-N and the second electrode 320-N in the monopolar type. The first electrode 310-N and the second electrode 320-N are connected to a high frequency power generation unit in the monopolar-type, and thus the delay may be reduced during an alternating output.

In this case, when the alternating output of a monopolar-type and a monopolar-type is performed, the separation of the first high frequency power generation unit 110 and the second high frequency power generation unit 120 is additionally applied to prevent interference effects between a plurality of electrodes in close proximity within the electrode unit 300.

The smoothly alternating output between a bipolar-type and a monopolar-type is performed by the switching circuit 130.

It is assumed that the bipolar-type current is output after the monopolar-type current is output. First, when the monopolar-type current is output, an electrode of a first polarity of the first high frequency power generation unit 110 may be connected to the first electrode 310-N and the second electrode 320-N, and an electrode of a second polarity of the first high frequency power generation unit 110, which is opposite to the first polarity of the first high frequency power generation unit 110, may be connected to an NE pad, through the switching operation control of the switching circuit 130. Accordingly, under control of the control unit 140, a current flows between the NE pad and the first electrode 310-N and the second electrode 320-N. Afterward, when the bipolar-type current is output, the electrode of the second polarity of the first high frequency power generation unit 110 may be connected to the second electrode 320-N through the switching operation control of the switching circuit 130 while being connected to the NE pad. Accordingly, under control of the control unit 140, a current flows between the first electrode 310-N and the second electrode 320-N. On the other hand, when the monopolar-type current is output after the bipolar-type current is output, the control unit 140 may control the above process in reverse.

The electrode unit 300 may be configured to directly or indirectly output a current to the skin .

The skin may refer to skin that performs high frequency irradiation for tightening, acne, or pigmentation.

The electrode unit 300 consists of one or more first electrodes and one or more second electrodes.

When a current of the same polarity is applied to the first electrode 310-N and the second electrode 320-N of the electrode unit 300, the monopolar-type is formed. When currents having different polarities are applied to the first electrode 310-N and the second electrode 320-N, the bipolar-type is formed.

The monopolar-type may refer to a type in which a single polarity is output from an electrode.

The monopolar-type may refer to the output type of a plurality of electrodes that operate to circulate a high frequency current in a ground electrode in contact with skin at a separate location when the high frequency current is delivered from an electrode.

The bipolar-type may refer to a type in which different polarities are output from electrodes.

The bipolar-type may mean an output form of a plurality of electrodes that operates to circulate a high frequency current in a surrounding cathode, when the high frequency current is delivered from an anode among a plurality of electrodes to skin.

The bipolar-type has two electrodes placed at narrow intervals, and thus the affected range may be reduced by using a high frequency current. Accordingly, except for a specific portion of high frequency irradiation, the remaining portions may not be affected by the high frequency current.

The inventive concept may improve skin tightening by using the monopolar-type current and the bipolar-type current together. The descriptions of a role of each type in a process of skin tightening high frequency irradiation will be described below.

The electrode unit 300 may be generated as an invasive microneedle or a plurality of non-invasive electrodes.

According to an embodiment of the inventive concept, when the invasive electrode is formed with a plurality of microneedles, the thickness of each microneedle may be within a range of 0.15 mm to 1.0 mm. However, it is most preferable that the thickness of each microneedle is within the range of 0.15 mm to 0.35 mm.

In this case, the current provided by the first and second high frequency power generation units 110 and 120 may be used to increase the temperature of an active area (i.e., a target area), which is electromagnetically energized, below a skin surface through a microneedle to a level at which tissue is heated. The microneedle may be manufactured in various shapes such as a non-insulated needle, a needle with an active area formed at a distal end, and a needle including a plurality of active areas at the same specific location.

Besides, even when the electrode unit 300 is composed of a plurality of non-invasive electrodes, the electrode unit 300 is used to increase the temperature of the target area to a level where tissue is heated, as in the case that the electrode unit 300 is composed of microneedles.

In this case, it is preferable that a size of each of the plurality of electrodes is determined as being within a range of 0.16 mm to 3 cm. It is most preferable that a size of each of the plurality of electrodes is determined as being within a range of 0.16 mm to 10 mm.

Moreover, a height of each of the plurality of electrodes may be generated within a range **from 0 mm** to 50 mm. It is preferable that the height of each of the plurality of electrodes is generated within a range of 0 mm to 10 mm.

In the meantime, according to an embodiment, a film provided to cover the electrode unit 300 may be further included.

In this case, the area of the film may be generated within the range of 0.25 cm² to 25 cm².

On the basis of the power generated from at least one of the first high frequency power generation unit 110 and the second high frequency power generation unit 120, the plurality of electrodes output monopolar-type currents by delivering the current of two electrodes to the electrode unit 300, or the plurality of electrodes output bipolar-type currents by delivering currents having different polarities to the first electrode 310-N and the second electrode 320-N, under control of the control unit 140.

In detail, when the control unit 140 outputs the bipolar-type current by using the first high frequency power generation unit 110 (i.e., the case of outputting currents of different polarities to the first electrode 310-N and the second electrode 320-N), alternating current may be applied.

In this case, only the first high frequency power generation unit 110 is basically operated, and the second high frequency power generation unit 120 may be additionally operated to prevent interference effects between a plurality of electrodes in close proximity within the electrode unit 300.

Moreover, when the control unit 140 outputs the bipolar-type current by using the second high frequency power generation unit 120 (i.e., the case of outputting currents of different polarities to the first electrode 310-N and the second electrode 320-N), alternating current may be applied.

In this case, only the first high frequency power generation unit 110 may be basically operated, and the second high frequency power generation unit 120 may be additionally operated for the purpose.

In this case, even when the control unit 140 outputs the monopolar-type current, only the first high frequency power generation unit 110 may be basically operated, and the second high frequency power generation unit 120 may be additionally operated for the purpose.

In this case, the current of the same polarity may be output to the first electrode 310-N and the second electrode 320-N, and alternating current may be applied.

In the meantime, it is a need for a configuration of an NE pad connected to a polarity different from the polarity applied to the electrode unit 300 when the electrode unit 300 outputs the monopolar-type current.

Meanwhile, when the control unit 140 outputs each type of current, the relay 132 included in the switching circuit 130 may be operated.

When the distributor 131 and the relay 132 included in the switching circuit 130 are operated, an interval of up to 500 ms may occur between outputs.

In the meantime, the monopolar-type current and the bipolar-type current may be output under control of the control unit 140. The control unit 140 may select the same or different frequencies for each output.

Furthermore, the control unit 140 may have the following output pattern in which the monopolar-type current and the bipolar-type current are combined and output simultaneously or sequentially in an output setting pattern.

In other words, under control of the control unit 140, the monopolar-type current and the bipolar-type current are output simultaneously or sequentially in the output setting pattern, which is selected by the user or automatically set.

In this case, the monopolar-type current refers to a current that increases a temperature of a current supply area. The bipolar-type current refers to a current that locally increases the temperature of a local area by supplying energy to the local area.

For example, when the monopolar-type current and the bipolar-type current are output simultaneously, the needle electrode may output the bipolar-type current, and the monopolar-type current may be output to a skin surface from a needle tip attachment surface, which is a surface, on which a needle is coupled, to a needle tip.

Besides, patterns output in the output setting pattern include a monopolar-bipolar pattern MB, a bipolar-monopolar pattern BM, a monopolar-bipolar-monopolar pattern MBM, a bipolar-monopolar-bipolar pattern BMB, and the like.

Meanwhile, the control unit 140 may have the following pattern in which monopolar-type and bipolar-type currents or bipolar-type and monopolar-type currents are output sequentially.

For example, patterns output continuously include the monopolar-monopolar pattern MM or the bipolar-bipolar pattern BB.

In other words, the monopolar-monopolar pattern MM refers to a pattern in which the monopolar-type current is output under a first condition, and then the monopolar-type current is continuously output under a second condition.

Moreover, the bipolar-bipolar pattern BB refers to a pattern in which the bipolar-type current is output under the first condition, and then the bipolar-type current is continuously output under the second condition.

Among the patterns, in the case of the monopolar-monopolar pattern MM, interference effects between a plurality of electrodes adjacent to each other within the electrode unit 300 may be prevented by additionally configuring the second high frequency power generation unit 120. Accordingly, the current of the same polarity may be uniformly output for each electrode, thereby maximizing monopolar-type effects.

Moreover, the control unit 140 may allow the electrode unit 300 to output the monopolar-type current based on the user's input operation, and then may allow the electrode unit 300 to output the bipolar-type current after a predetermined time elapses.

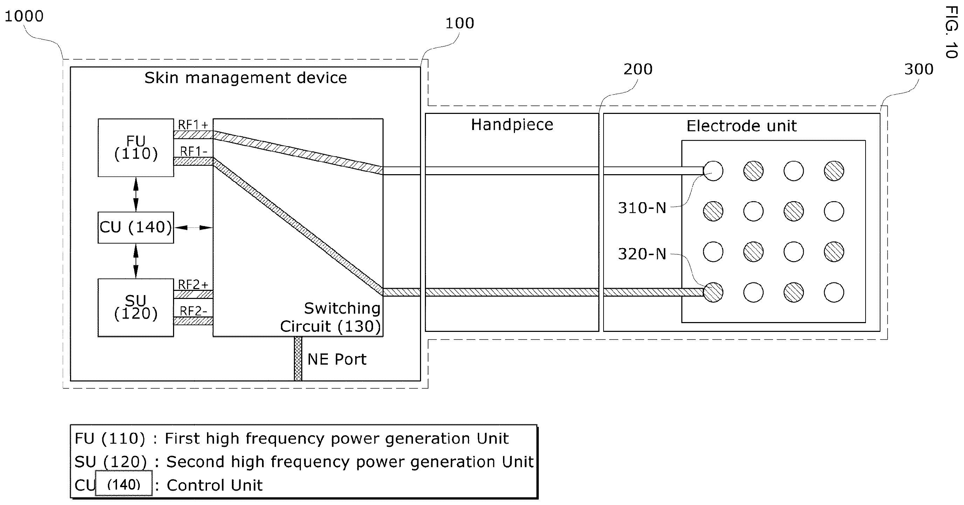

FIG. 10 is a block diagram for describing a case where a power output mode in the system shown in FIG. 1 corresponds to a bipolar-type, and the system shown in FIG. 1 includes the high frequency current output device 1000.

The high frequency current output device 1000 includes the skin treatment device 100, the handpiece 200, and the electrode unit 300.

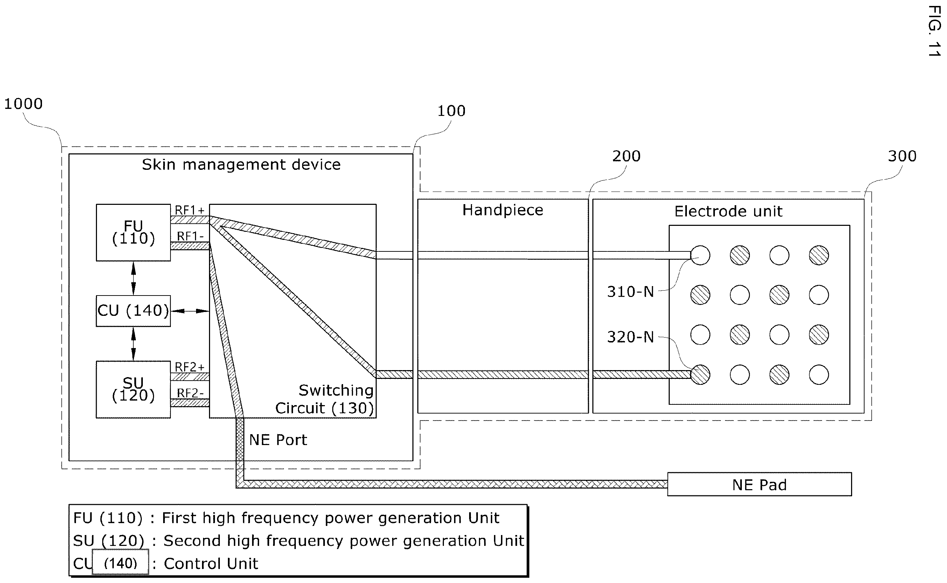

FIG. 11 is a block diagram for describing a case where a power output mode in the system shown in FIG. 1 corresponds to a monopolar-type.

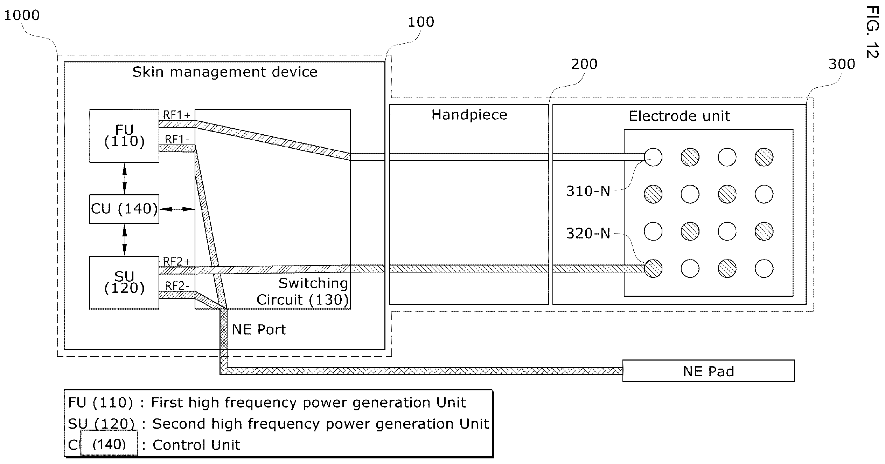

FIG. 12 is a block diagram illustrating a case where a power output mode in the system shown in FIG. 1 corresponds to a monopolar-type and the second high frequency power generation unit 120 is additionally operated when the first high frequency power generation unit 110 is operated.

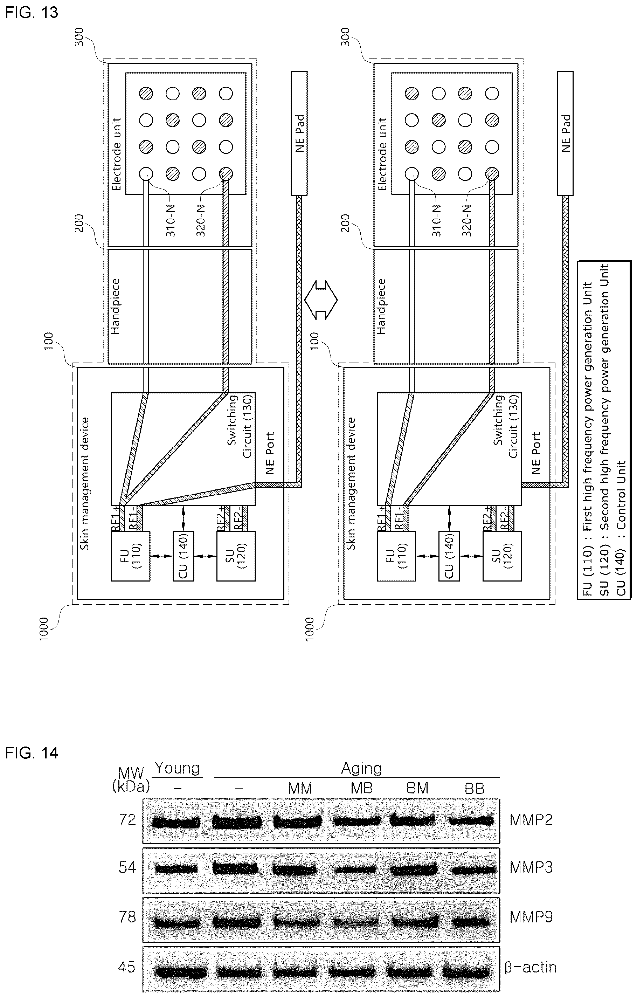

FIG. 13 is a block diagram for describing a case where a power output mode in the system shown in FIG. 1 continuously outputs a monopolar-type current and a bipolar-type current.

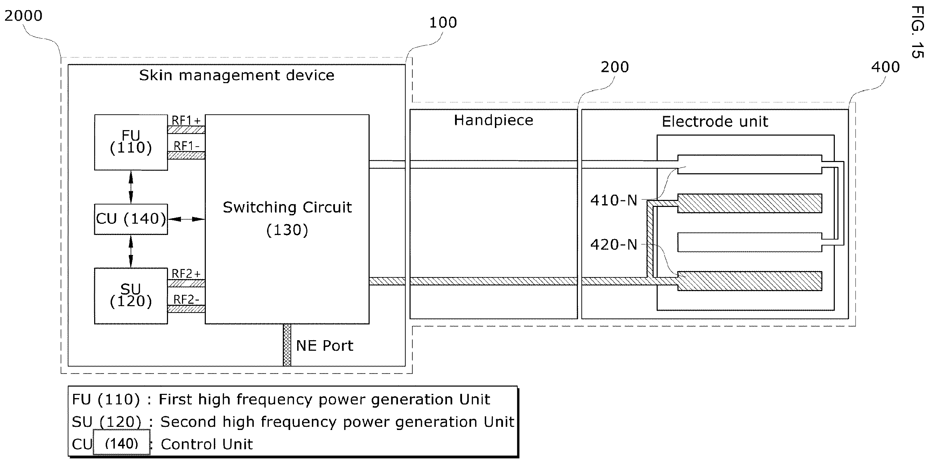

FIG. 15 is a block diagram of a system including a high frequency current output device 2000, according to another embodiment of the inventive concept. The system includes the skin treatment device 100, the handpiece 200, an electrode unit 400, and the high frequency current output device 2000.

The skin treatment device 100 includes the first high frequency power generation unit 110, the second high frequency power generation unit 120, the switching circuit 130, and the control unit 140.

In this case, as in the high frequency current output device 1000 according to an embodiment shown in FIG. 1, only the first high frequency power generation unit 110 is basically operated in the skin treatment device 100, and the second high frequency power generation unit 120 is additionally operated to prevent interference effects between a plurality of electrodes in close proximity within the electrode unit 300.

Moreover, the electrode unit 400 includes a plurality of first electrodes 410-1 to 410-N and a plurality of second electrodes 420-1 to 420-N.

Compared to the high frequency current output device 1000 according to an embodiment of the inventive concept shown in FIG. 1, in the high frequency current output device 2000 according to another embodiment of the inventive concept shown in FIG. 15, all other components of device 2000 are the same except for the electrode unit 400.

That is, the first electrode 410-N and the second electrode 420-N in the electrode unit 400 according to another embodiment of the inventive concept are film-type electrodes (FPCB), and the first electrode 410-N and the second electrode 420-N are electrically insulated at different locations on the FPCB, respectively.

In addition, there are the plurality of first electrodes 410-N, and there are the plurality of second electrodes 420-N. The first electrodes 410-N and the second electrodes 420-N are arranged in a plate shape such as a rectangle, right triangle, or isosceles triangle such that the first electrodes 410-N and the second electrodes 420-N do not overlap each other and electrically connected to each other.

This is to prepare for a leakage current between electrodes that occurs when the electrodes are placed alternately, because the film-type electrode is a thin film,

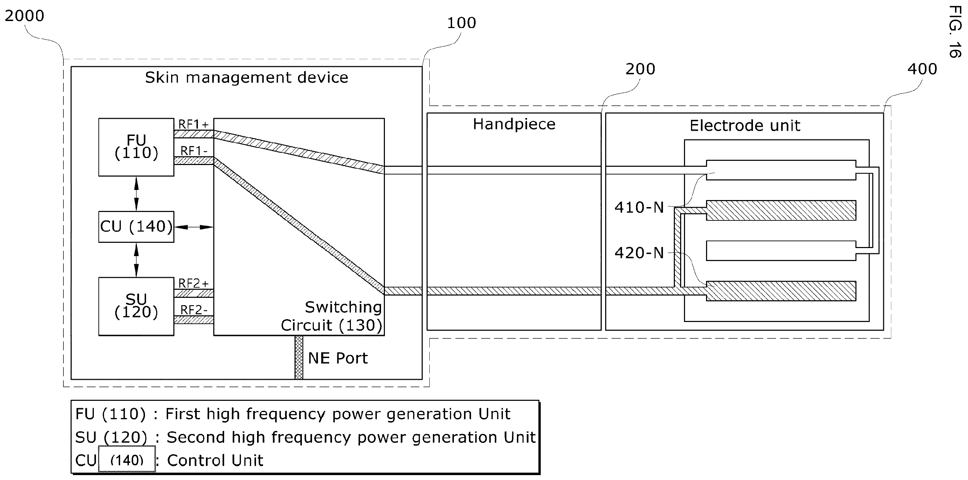

FIG. 16 is a block diagram for describing a case where a power output mode in the system shown in FIG. 15 corresponds to a bipolar-type. The system includes the skin treatment device 100, the handpiece 200, and the electrode unit 400.

Compared to the high frequency current output device 1000 according to an embodiment of the inventive concept shown in FIG. 10, in the high frequency current output device 2000 according to another embodiment of the inventive concept shown in FIG. 16, all other components of device 2000 are the same except for the electrode unit 400. Accordingly, an operation of the high frequency current output device 1000 according to an embodiment of the inventive concept in FIG. 10 may be applied to the descriptions of specific operations to be described later.

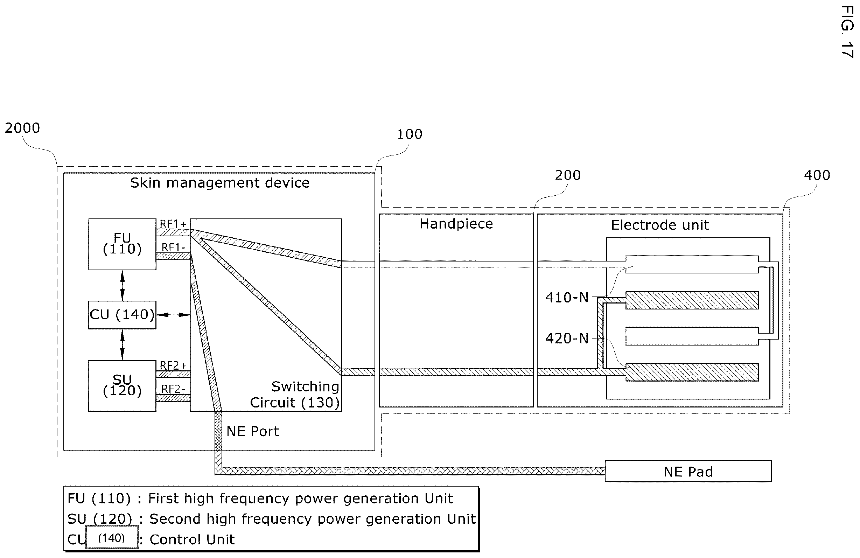

FIG. 17 is a block diagram for describing a case where a power output mode in the system shown in FIG. 15 corresponds to a monopolar-type. The system includes the skin treatment device 100, the handpiece 200, and the electrode unit 400.

Compared to the high frequency current output device 1000 according to an embodiment of the inventive concept shown in FIG. 11, in the high frequency current output device 2000 according to another embodiment of the inventive concept shown in FIG. 17, all other components of device 2000 are the same except for the electrode unit 400. Accordingly, an operation of the high frequency current output device 1000 according to an embodiment of the inventive concept in FIG. 111 may be applied to the descriptions of specific operations to be described later.

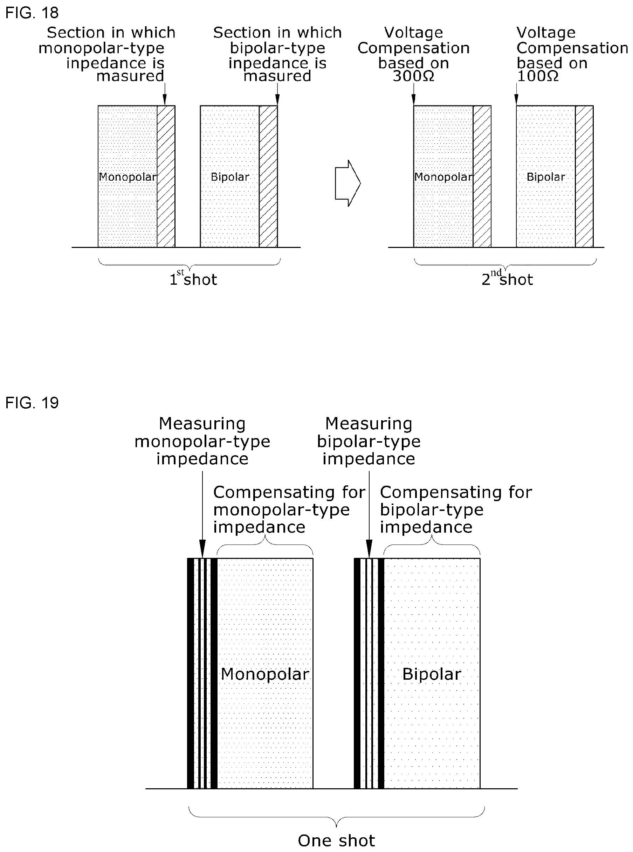

FIG. 18 is a conceptual diagram for describing a first embodiment of an impedance matching operation when a power output mode corresponds to the mono-bi pattern MB in the system shown in FIGS. 1 and 15.

FIG. 19 is a conceptual diagram for describing a second embodiment of an impedance matching operation when a power output mode corresponds to the mono-bi pattern MB in the system shown in FIGS. 1 and 15.

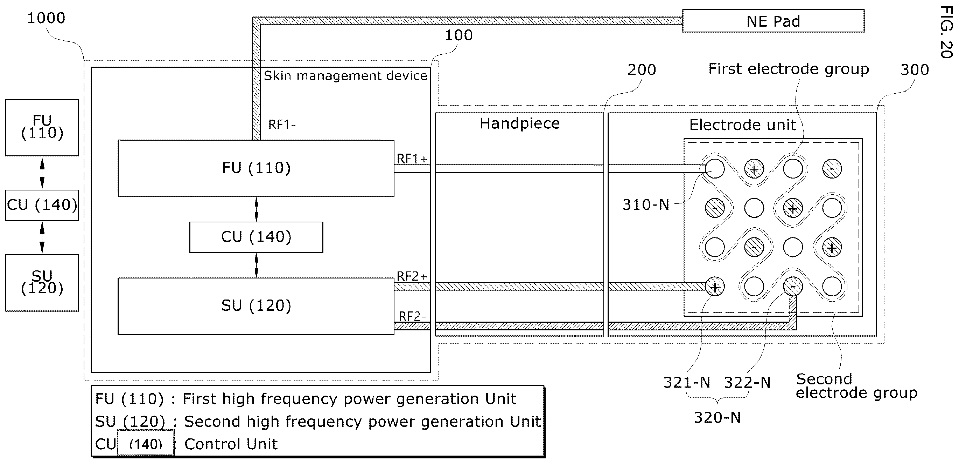

FIG. 20 is a block diagram of a system including a high frequency current output device 1000, according to still another embodiment of the inventive concept. The system includes the skin treatment device 100, the handpiece 200, the electrode unit 300, and an NE pad.

As shown in FIG. 10, the skin treatment device 100, the handpiece 200, and the electrode unit 300 are electrically connected to each other to continuously output a monopolar-type current and a bipolar-type current. The skin treatment device 100 includes the first and second high frequency power generation units 110 and 120 and the switching circuit 130.

Furthermore, when viewed from above the top, the first electrode 310-N and the second electrode 320-N in the electrode unit 300 are placed in a zigzag shape. In a cross-section, as illustrated in the lower right part of FIG. 1, the first electrode 310-N and the second electrode 320-N are electrically connected to different inner layers of a PCB stack, which are insulated from each other, respectively.

As such, the first electrode 310-N and the second electrode 320-N are placed in a zigzag shape to supply a current to skin, and are connected to different layers insulated from each other to supply a current.

Additionally, in the electrode unit 300, a plurality of electrodes may be formed on the surface of a needle tip or may be formed in a needle type.

The control unit 140 receives currents of (+) polarity and (-) polarity from both the first and second high frequency power generation units 110 and 120, and supply the currents to the first and second electrodes 310-N and 320-N of the electrode unit 300 in the current output mode.

In other words, in the current output mode, electrical connections to the first and second electrodes 310-N and 320-N of the electrode unit 300 are different depending on a bipolar-type, a monopolar-type, a monopolar repeat pattern, a monopolar and bipolar continuous pattern.

First of all, as illustrated in FIG. 10, when the current output mode corresponds to the bipolar-type, the control unit 140 controls the switching circuit 130 such that different polarities are connected to the first electrode 310-N and the second electrode 320-N of the electrode unit 300, and bipolar-type output is possible by applying alternating current.

In this case, the control unit 140 does not consider electrical connections for RF2+, RF2- and NE pads.

Next, as illustrated in FIG. 11, when the current output mode corresponds to a monopolar-type, the control unit 140 may allow the switching circuit 130 to simultaneously connect the first electrode 310-N and the second electrode 320-N of the electrode unit 300, and may allow a high frequency current RF1- of the first high frequency power generation unit 110 to be supplied to an NE pad through a separate port (NE Port) such that a monopolar-type output is possible.

For example, in the case where the electrode of the electrode unit 300 has (+) polarity when a high frequency current flows, the NE pad has (-) polarity. When the electrode of the electrode unit 300 has (-) polarity, the NE pad has (+) polarity. Accordingly, the current flows into a human body.

In this case, the control unit 140 does not consider electrical connections for RF2+ and RF2-.

Next, as illustrated in FIG. 12, in the case where the second high frequency power generation unit 120 is additionally operated as the first high frequency power generation unit 110 is operated, when the current output mode corresponds to the monopolar repeat pattern, the control unit 140 allows the switching circuit 130 to connect one polarity from the first high frequency power generation unit 110 to the first electrode 310-N of the electrode unit 300, and allows the switching circuit 130 to connect the same polarity as a polarity, which is connected from the second high frequency power generation unit 120 to the first high frequency power generation unit 110, to the second electrode 320-N.

Moreover, under control of the control unit 140, the high frequency current RF1- of the first high frequency power generation unit 110 is supplied to the NE pad through a separate port (NE Port) such that the monopolar-type output is possible.

Furthermore, the control unit 140 may allow the switching circuit 130 to connect the same polarity to the second electrode 320-N of the electrode unit 300, and may allow a high frequency current RF2- of the second high frequency power generation unit 120 to be supplied to an NE pad through a separate port (NE Port) such that a monopolar-type output is possible.

In this case, the control unit 140 may basically drive the first high frequency power generation unit 110 and may additionally drive the second high frequency power generation unit 120 such that the monopolar repeat pattern is possible.

As such, the second high frequency power generation unit 120 may be additionally operated, thereby preventing interference effects between a plurality of electrodes in close proximity within the electrode unit 300, and supplying a uniform current to a plurality of electrodes.

Next, as illustrated in FIG. 13, when the current output mode corresponds to the monopolar and bipolar continuous pattern, the control unit 140 may perform a continuous output operation while quickly switching between monopolar-type and bipolar-type connections, by mixing a case of a monopolar-type alternating output and a case of a bipolar-type.

In other words, in the case of the monopolar-type alternating output, when all electrodes have the same polarity as each other, NE pads have opposite polarities to each other. In the case of the bipolar-type, an NE pad is not electrically connected to a separate port (NE Port), and the first electrode 310-N and the second electrode 320-N of the electrode unit 300 cross each other and have different polarities.

In this way, the control unit 140 may sequentially perform outputs of the first high frequency power generation unit 110 and the second high frequency power generation unit 120 through the switching circuit 130 such that a continuous pattern of bipolar-type and monopolar-type is possible.

In all of the embodiments described in FIGS. 10 to 13, the handpiece 200, which is a part that a doctor holds, is located between the skin treatment device 100 and the electrode unit 300 to operate as an intermediate medium, and is moved while being in contact with a subject's skin to change a target point.

In general, the density of collagen and elastin fibers in aged skin is lower than in young skin. The application of electrical energy (RF) has a significant impact on the formation of collagen and elastin, thereby usefully applying rejuvenation such as tightening and lifting.

In particular, the monopolar-bipolar pattern MB has been experimentally shown to significantly increase the density of collagen and elastin fibers compared to other patterns.

According to an embodiment of the inventive concept, in the case of the monopolar-bipolar pattern MB in which the monopolar-type output is irradiated and then the bipolar-type output is irradiated, first of all, energy may be delivered to the lower dermal layer of skin in a monopolar type.

Next, when the bipolar-type output is irradiated immediately afterwards, because the resistance of skin is low, energy having high dense may be evenly delivered to a skin layer to which the monopolar-type output is delivered.

In other words, a portion having a high temperature has low electrical resistance due to electrical characteristics. Accordingly, the resistance is relatively low in a portion where a skin temperature is increased due to the monopolar-type output, allowing electricity to flow easily.

In general, as the temperature of human tissue increases, impedance, which is a resistance component, decreases by 1.5% to 2%, and the electrical conductivity increases in inverse proportion to the impedance. The principle of selectively finding a path where a current flows better is applied to high frequencies.

In other words, the monopolar-type output, which is irradiated first, generally increases the overall temperature of a skin area. The bipolar-type output irradiated afterward supplies energy locally to the corresponding skin area in a state where a temperature is increased, concentrating ablation effects on skin tissues. The principle of advantages of an output of the monopolar-bipolar pattern MB compared to a bipolar-type single output is described in detail as follows.

When a bipolar-type current is output immediately after outputting a monopolar-type current, a current may flow through fibrous septae deeper than when the bipolar-type current is output alone, due to the characteristics of a high frequency current.

Because everything surrounding fat tissue is fibrous septae made of collagen, it may be expected that a bipolar-type single output current may not affect a fat layer in general. In a state of pre-heating a skin area with the monopolar-type output, a current may flow deeper into lower dermis or even a shallow subcutaneous fat layer, causing heating at the corresponding location.

For this reason, when energy is applied by using an output of the monopolar-bipolar pattern MB, effective energy may be applied deeper than when the bipolar-type current is output alone.

In more detail, many elastic fibers called oxytalan fibers are distributed in the upper dermis layer. These fibers give elasticity to the skin and help prevent fine wrinkles.

When electrical (RF) energy having a vertical waveform is delivered by monopolar-type irradiation, the overall temperature of tissues increases.

When the bipolar-type output is irradiated immediately afterward, the electrical (RF) energy is delivered to a place, at which a current flows easily, for example, not only to the epidermis but also to the upper dermis, due to the lowered resistance and increased conductivity.

In this way, collagen and elastin may be formed in large quantities by intensively applying electrical (RF) energy to the upper dermis layer, which is important for elastin production.

Here, a predetermined time may be determined depending on the temperature of the skin.

In other words, the control unit 140 may determine the predetermined time based on a temperature change in the skin after the electrode unit 300 outputs a monopolar-type current.

In particular, when a monopolar-type current is applied to the skin, a temperature of the skin may increase.

Afterward, when the current type of the electrode unit 300 is changed from a monopolar-type to a bipolar-type, a current is not supplied to the skin while when the current type is changed, and thus the temperature of the skin may drop.

The control unit 140 may determine the predetermined time and may allow the bipolar-type current to be applied to the skin within the predetermined time so as to prevent the temperature of the skin from falling below a specific temperature during the predetermined time.

When continuous irradiation of the monopolar-bipolar pattern MB or the bipolar-monopolar pattern BM is possible within little or very short relay time during a thermal damage time (TDT), which is a time required to thermally damage the desired tissue without thermal damage to the surrounding area, the above-described high frequency irradiation effects may be achieved.

In the meantime, the control unit 140 may allow the electrode unit 300 to sequentially output a monopolar-type current or a bipolar-type current and then to output the monopolar-type current or the bipolar-type current based on a user's manual operation.

That is, the control unit 140 may allow the electrode unit 300 to output a monopolar-type current and then to output the bipolar-type current (i.e., the monopolar-bipolar pattern MB), may allow the electrode unit 300 to output the monopolar-type current and then to output the monopolar-type current (i.e., the monopolar-monopolar pattern MM), may allow the electrode unit 300 to output the bipolar-type current and then to output the monopolar-type current (i.e., the bipolar-monopolar pattern BM), or may allow the electrode unit 300 to output the bipolar-type current and then to output the bipolar-type current (i.e., the bipolar-bipolar pattern BB).

As a result of the actual experiment, the mono-bi pattern MB, the mono-mono pattern MM, the bi-mono pattern BM, and the bi-bi pattern BB significantly increase collagen and elastin fibers, compared to only the conventional monopolar-type or bipolar-type. Among the above-described current output options, in particular, the mono-bi pattern MB was found to have a greater effect on a decrease in CD80 (M1 marker), an increase in CD206 (M2 marker), a decrease in TNF-α and increase in IL-10 in skin, a decrease in RAGE and NF-_{κ}B in aged skin, an increase in collagen, an increase of fibrillin (FBN), an increase in collagen fibers, and an increase in elastin fibers.

In particular, in the case of the bi-mono pattern BM, in which the bipolar-type current is first applied and then the monopolar-type current is applied, electrical (RF) energy is delivered to the upper layer of skin better than the mono-bi pattern MB.

For this reason, because a current flows better as a temperature is higher, the electrical conductivity is increased by the monopolar-type current applied immediately afterward, by supplying a bipolar-type current of which the electrical (RF) energy is shallowly delivered in a horizontal direction. Accordingly, the electrical (RF) energy may be delivered in a direction in which the current flows better.

This pattern may be a pattern that is useful when pigmentation, redness, or pores are improved by targeting the upper layer of skin.

In this case, when the electrical (RF) energy is applied in the mono-bi pattern MB or the bi-mono pattern BM, impedance measured in a monopolar-type is compensated for (matched) in the monopolar-type, and impedance measured in a bipolar-type is compensated for (matched) in the bipolar-type.

Impedance matching means a compensation method of reducing reflection caused by impedance of two different connection terminals when an output terminal is connected to an input terminal.

In the inventive concept, monopolar-type impedance and bipolar-type impedance are compensated for, and high frequency energy suitable for each type of high frequency irradiation of a patient may be applied such that the high frequency irradiation effect is appropriate for the type.

This is described in detail as follows.

There is a difference in a length, at which electricity is circulated, between a monopolar-type and a bipolar-type.

Accordingly, the monopolar-type has higher impedance on average than the bipolar-type.

The monopolar-type impedance is about 300 Ω on average, and the bipolar-type impedance is about 100 Ω on average.

Because the resistance is high when an impedance value is high, more energy needs to be supplied.

An average impedance value of a monopolar-type and a bipolar-type is about 200 Ω. Accordingly, when impedance matching is performed by using the average value, an issue in which energy is little consumed in the monopolar-type, and a lot of energy is consumed in the bipolar-type may occur in impedance matching.

However, the method of matching impedance so far may include measuring impedance at the previous stage of a shot and compensating for impedance for the corresponding shot, or measuring impedance at the end of a shot and compensating for impedance for the next shot.

However, when a monopolar-bipolar pattern or a bipolar-monopolar pattern is delivered in one shot, both the monopolar-type and the bipolar-type need to be applied in one shot, and thus it may be difficult to expect the intended effect of high frequency irradiation because a time point of measuring impedance is difficult from a time point of compensating for impedance.

Accordingly, in an embodiment of the inventive concept, the impedance is matched in the following method.

First, the impedance of tissues may be measured at the previous stage of the shot, and the set power may be supplied within the duration of the set value.

Moreover, real-time impedance may be measured and compensated for in units of 1 to 2 ms, and impedance may be detected and compensated for in real time even while the electrical (RF) energy is being applied.

Furthermore, when impedance is measured and compensated for in one shot (e.g., a mono-bi pattern), the monopolar-type impedance is measured at the end of a monopolar-type shot to supply power corresponding to the measured impedance, and then the bipolar-type impedance is measured at the end of a bipolar-type shot to supply power corresponding to the measured impedance.

Besides, as illustrated in FIG. 18, the monopolar-type impedance and the bipolar-type impedance are measured at the first shot, and monopolar-type impedance matching and bipolar-type impedance matching are performed at the second shot.

That is, in the case of the mono-bi pattern MB, after the monopolar-type impedance and the bipolar-type impedance are measured at the first shot, the power of the second shot is adjusted by matching an impedance value measured at the first shot with an impedance value measured at the second shot.

FIG. 18 illustrates an impedance matching operation in which impedance is measured at the previous stage of a shot in the case of the mono-bi pattern MB. However, a case of the bi-mono pattern BM is also possible.

In another embodiment of the inventive concept, the impedance is matched in the following method.

In an embodiment, impedance is measured before or after RF output, and an RF output condition is compensated for and adjusted by using the measured impedance. For example, the impedance may be measured before the RF output (the first detailed shot within an output shot, for example, a monopolar shot) and then impedance of the first detailed shot may be compensated for. After the RF output (the first detailed shot within an output shot, for example, a monopolar shot) of a specific shot, impedance may be measured and an output condition for the next shot (the second detailed shot within the output shot, for example, a bipolar shot) may be adjusted.

In detail, as illustrated in FIG. 19, when impedance is measured and compensated for at the previous stage of one shot (e.g., a mono-bi pattern), the monopolar-type impedance is measured at the previous stage of a monopolar-type shot to supply power corresponding to the measured impedance, and then the bipolar-type impedance is measured at the previous stage of a bipolar-type shot to supply power corresponding to the measured impedance.

Also, the monopolar-type impedance matching and the bipolar-type impedance matching are performed in the second shot that immediately follows.

For another example, the control unit 140 may allow the electrode unit 300 to output a current in the mono-bi-mono pattern MBM.

In this pattern, the electrical (RF) energy is delivered in a deep vertical direction → shallow horizontal direction → deep vertical direction. In this case, the current flows deep and abundantly under skin and thus affects fat reduction and collagen production.

In this case, the overall temperature of epidermis and dermis may be increased due to the monopolar-type output thus applied first. Accordingly, a resistance value of skin may be lowered such that electricity flows easily.

A portion of the skin having the highest impedance is the epidermis. Because the monopolar-type output first raises a skin temperature, an environment may be created such that a current flows efficiently into the epidermis.

Moreover, the current flows locally through an epidermal layer having lowered resistance due to a bipolar-type output thus second applied, and thus the temperature of the epidermal layer is increased. Because the resistance decreases when the temperature of the epidermal layer increases, the energy of the monopolar-type output to be applied next time may penetrate the skin deeply.

Furthermore, the impedance of the skin is lowered due to the monopolar-type output thus third applied, and thus an environment may be created such that an effective current flows deeper. Accordingly, fat may be reduced by flowing current not only into the dermis but also into the fat layer.

In other words, fat is usually connected with a septum. Because the septum has higher electrical conductivity than the fat, the third monopolar-type allows a current to flow into the entire fat layer through the septum.

Accordingly, when the fat layer is stimulated, stem cell secretion may be active and thus the fat layer may be reduced. Also, skin elasticity may be improved by producing collagen and elastin in the dermal layer.

Accordingly, this pattern may be a pattern useful for a patient whose skin has progressed in aging but who has a lot of fat under the skin of a face.

For another example, the control unit 140 may allow the electrode unit 300 to output a current in the bi-mono-bi pattern BMB.

The pattern affects melanin by delivering effective electrical (RF) energy to epidermis without causing cell death. Accordingly, experimental results useful for improving freckles have recently been published in a paper.

Accordingly, this pattern improves freckles by delivering powerful electrical (RF) energy to the epidermal layer, and strengthens basement membrane. Accordingly, this pattern may be a pattern useful to prevent freckles from recurring.

In the meantime, a mono-bi pattern and a bi-mono pattern may be output sequentially.

Moreover, under control of the control unit 140, a bipolar-type current may be output after a single monopolar-type current is output or a plurality of monopolar-type currents are output.

For example, the control unit 140 may allow the electrode unit 300 to output a current in a mono-mono-bi pattern in which a monopolar-type current is output after a monopolar-type current is output and then a bipolar-type current is output.

Furthermore, the control unit 140 may allow the electrode unit 300 to output a current in a mono-bi-bi pattern in which a bipolar-type current is continuously output after a monopolar-type current is output.

Moreover, after the single bipolar-type current is output or the plurality of bipolar-type currents are output, a monopolar-type current may be output.

For example, the control unit 140 may allow the electrode unit 300 to output a current in a bi-bi-mono pattern in which a bipolar-type current is output after a bipolar-type current is output, and then a monopolar-type current is output.

Furthermore, the control unit 140 may allow the electrode unit 300 to output a current in a bi-mono-mono pattern that a monopolar-type current is continuously output after a bipolar-type current is output.

In the meantime, in the pattern thus output, it is important that a monopolar-type current and a bipolar-type current are alternately used. The output form, such as the order of types, is not limited thereto.

The control unit 140 may allow the electrode unit 300 to output the bipolar-type current based on the user's input operation, and then may allow the electrode unit 300 to output the monopolar-type current after a predetermined time elapses.

Here, the predetermined time may be determined as a time of 500 ms or less.

According to another embodiment of the inventive concept, the control unit 140 may determine that the predetermined time is within a range of 500 ms.

In the meantime, when a plurality of electrodes in the electrode unit 300 is formed in the first electrode 310-N and the second electrode 320-N in a plurality of needle type, high frequency irradiation may also be performed while a pattern that alternatively changes a bipolar-type current and a monopolar-type current is output in a process of inserting a needle

Meanwhile, the control unit 140 may determine monopolar-type output energy and bipolar-type output energy within a range of 2 watts to 400 watts.

In addition, the control unit 140 may determine monopolar-type pulse duration and bipolar-type pulse duration within a range of 10 ms to 9000 ms. It is preferable that the control unit 140 determines monopolar-type pulse duration and bipolar-type pulse duration within a range of 10 ms to 990 ms.

The control unit 140 may determine the frequency of a current generated by each of the first high frequency power generation unit 110 and the second high frequency power generation unit 120 within a range of 0.3 MHz to 67.8 MHz. It is preferable that the control unit 140 may determine that the frequency of the current is within a range of 0.5 MHz to 67.8 MHz.

Moreover, the control unit 140 may control a switching element 130 capable of delivering currents of two electrodes or different polarities to the electrode unit 300.

At least one component may be added or deleted to correspond to the performance of the components of the high frequency current output device 1000 shown in FIG. 1.

Furthermore, it will be easily understood by those skilled in the art that mutual locations of the components may be changed to correspond to the performance or structure of the system.

In the meantime, each component shown in FIG. 1 refers to software components and/or hardware components such as field programmable gate array (FPGA) and application specific integrated circuit (ASIC).

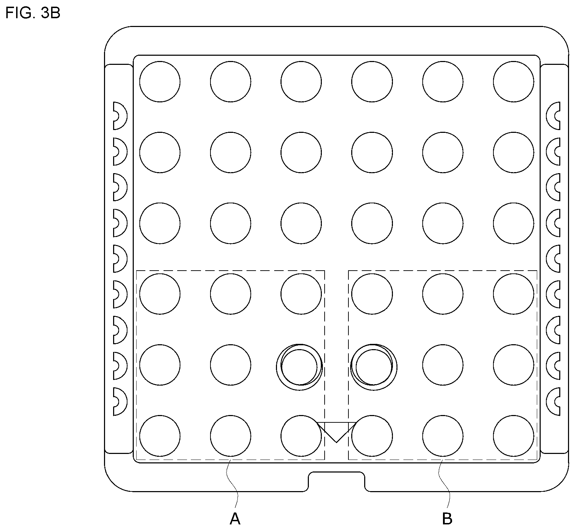

FIG. 3A is a vertical cross-sectional view of a plurality of electrodes, according to an embodiment of the inventive concept. FIG. 3B is a top plan view of a plurality of electrodes, according to an embodiment of the inventive concept.

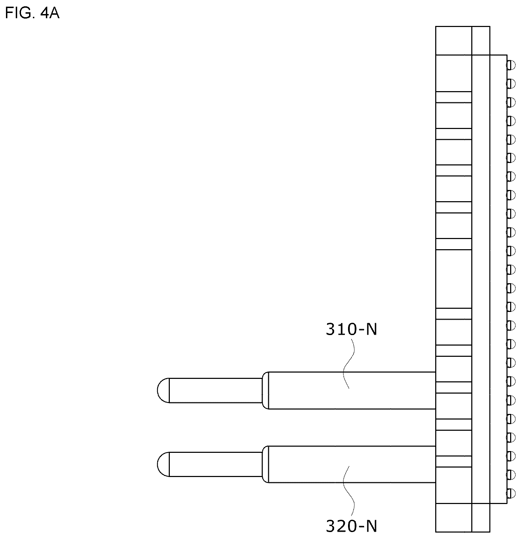

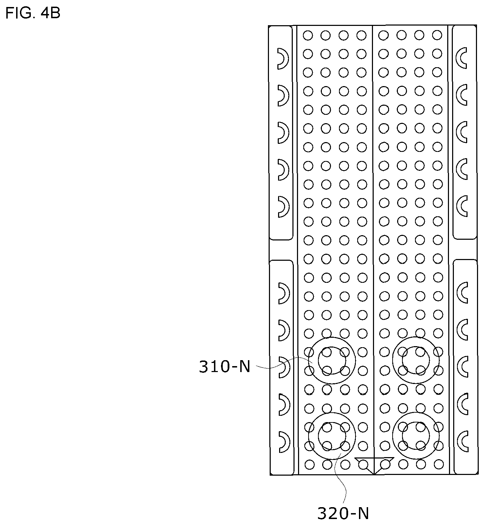

FIG. 4A is a vertical cross-sectional view when a plurality of electrodes are provided as non-invasive electrodes, according to an embodiment of the inventive concept. FIG. 4B is a top plan view of a plurality of electrodes in the case of FIG. 4A.

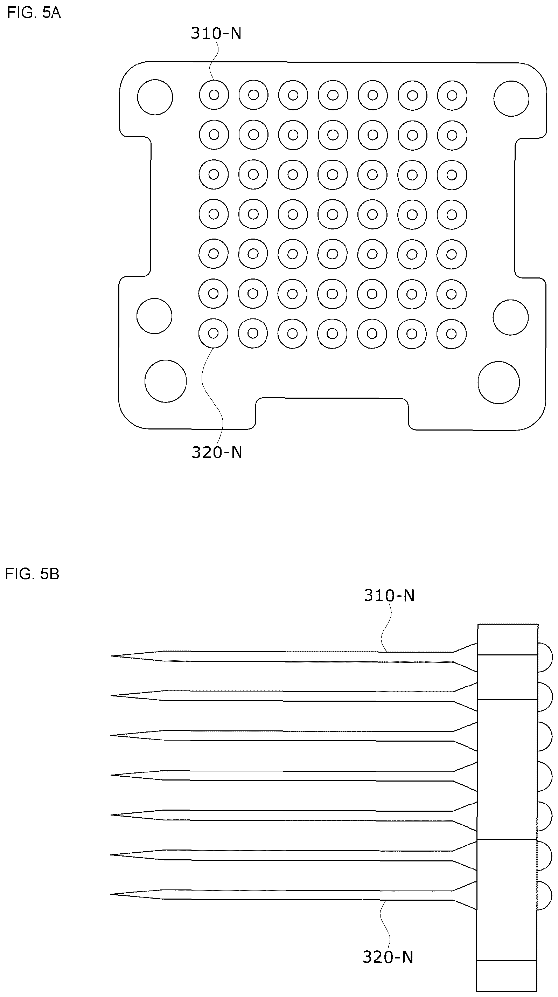

FIG. 5A is a top plan view of a plurality of electrodes provided as invasive electrodes, according to an embodiment of the inventive concept. FIG. 5B is a side view of a plurality of electrodes in the case of FIG. 5A.

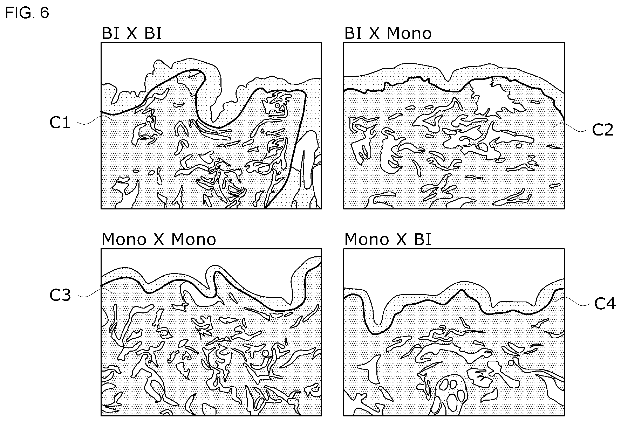

FIG. 6 is an image illustrating results of collagen production according to a monopolar-type current output or a bipolar-type current output, according to an embodiment of the inventive concept.

Referring to FIG. 6, in the case of the bi-bi pattern BB, in which a bipolar-type current is output after a bipolar-type current is output, the bi-mono pattern BM, in which a monopolar-type current is output after a bipolar-type current is output, the mono-mono pattern MM, in which a monopolar-type current is output after a monopolar-type current is output, and the mono-bi pattern MB, in which a bipolar-type current is output after a monopolar-type current is output, results of collagen production may be compared to each other. Here, an area of a collagen production result value of the bi-bi pattern BB may be C1; an area of a collagen production result value of the bi-mono pattern BM may be C2; an area of a collagen production result value of the mono-mono pattern MM may be C3; and, an area of a collagen production result value of the mono-bi pattern MB may be C4.

As a result of the tissue experiment as shown in FIG. 6, it may be identified that a high frequency output of the mono-bi pattern MB, which is a series of a monopolar-type current and a bipolar-type current, and the bi-mono pattern BM, which is a series of a bipolar-type current and a monopolar-type current, is the most effective in collagen production compared to a bipolar-type current or a monopolar-type current.

A facial skin is largely composed of three layers. An outer layer is epidermis; a dermis layer, which is rich in collagen, is under the epidermis; and, there is a complex network of collagen fibers called a subcutaneous layer (a fat layer) in the lower dermis.

Collagen present in the skin is denatured due to exposure to ultraviolet rays, family history, aging process, or the like, causing wrinkles to be formed. Accordingly, various treatment methods, which only act on each skin layer, have clinical limitations because they do not cause overall biochemical changes in collagen itself.

In fact, in clinical practice, to achieve clinical effects on all skin layers, the skin layer is irradiated first by using a bipolar (or monopolar) type current, and then the type is changed to use the monopolar (or bipolar) type current.

When a type is selected, an additional manipulation (task) is required to change from the monopolar-type to the bipolar-type or from the bipolar-type to the monopolar-type. Due to technical limitations, a device failed to be supplied to output monopolar-type and bipolar-type currents or bipolar-type and monopolar-type currents simultaneously or with a short relay time in one operation.

The device according to an embodiment of the inventive concept performs a continuous output in one pattern (MB, BM, MBM, BMB, MMB, BBM, or the like) obtained by combining a monopolar-type and a bipolar-type or a bipolar-type and a monopolar-type, thereby maximizing effects of high frequency irradiation.

Moreover, as shown by the dotted line in FIG. 3B, a plurality of electrodes within the electrode unit 300 may be classified into a monopolar-type electrode group (A) and a bipolar-type electrode group (B). The monopolar-type current and bipolar-type current may be output simultaneously for each group.

FIG. 3B illustrates that the plurality of electrodes is classified into four groups for ease of understanding, but the plurality of electrodes may be further subdivided to form sub-groups.

According to an embodiment of the inventive concept, it has been identified that collagen and collagen fibers are increased in both upper dermis and lower dermis when the monopolar-type output is first irradiated and then the bipolar-type output is subsequently irradiated, and it has been identified that this energy transfer pattern thermally acts on all layers of skin, causing overall biochemical changes in collagen.

Due to electrical characteristics, a place at a high temperature has low electrical resistance, and thus electricity flows easily.

When the monopolar-type energy is delivered to the lower dermis layer of skin, and then the bipolar-type output is irradiated immediately afterwards, energy having high density may be evenly delivered to a skin layer to which the monopolar-type output is delivered because the resistance is low.

When the bipolar-type output is first irradiated and the monopolar-type output is subsequently irradiated, an area where the bipolar-type output is delivered has low resistance, and thus electricity flows easily. The energy required for high frequency irradiation is delivered to the epidermis and upper dermis, including spots, capillaries, and pores.

In addition, because the monopolar-type high frequency energy, which is sequentially output after the bipolar-type high frequency energy is output, is delivered to the lower dermis, a synergistic treatment effect may be achieved due to the generation of deep heat.

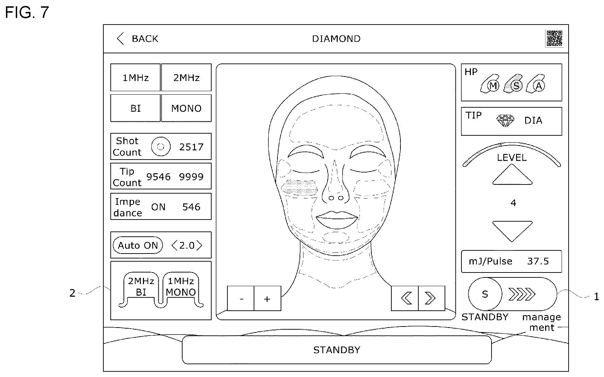

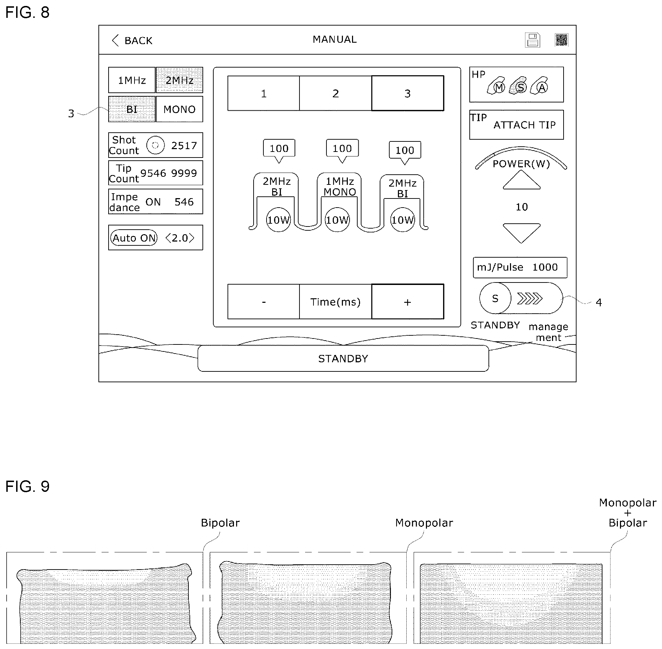

FIGS. 7 and 8 are diagrams showing a user interface provided by a high frequency current output device to control current outputs of a plurality of electrodes, according to an embodiment of the inventive concept.

As shown in FIG. 7, when a pattern output key 1 in a user interface is entered, a combination pattern of a bipolar-type current and a monopolar-type current is output, and then information indicating a pattern being output within a specific area 2 of the user interface may be displayed under control of the control unit 140.

In other words, a user may allow the high frequency current output device 1000 to output a current in various combination patterns of a monopolar-type and a bipolar-type through the user interface.

Furthermore, the user may select various patterns depending on a current output option, where a current is output, through the user interface of a device main body and may set monopolar-type and/or bipolar-type pulse duration, energy output intensity, or the like within the corresponding pattern, thereby receiving various high frequency irradiation effects with various types of patterns through adjusting these settings.

That is, as shown in FIG. 8, when a specific area 3 within the user interface is selected, the control unit 140 may provide the user with a setting window of the monopolar-bipolar (MB) pattern or the bipolar-monopolar (BM) pattern. When the pattern output key 1 is entered after a monopolar-bipolar pattern or a bipolar-monopolar pattern is set by the user through the settings window, various combination patterns of a monopolar-type and a bipolar-type set by the user may be output under control of the control unit 140.

FIG. 9 is a diagram showing a frequency heat transfer range when an electrode operates in a bipolar-type, operates in a monopolar-type, and operates in a monopolar-bipolar (MB) pattern, according to an embodiment of the inventive concept.

According to an embodiment of the inventive concept, a high frequency output device may output a new energy transfer pattern by changing a method of delivering energy and may induce an immediate reaction, in which skin contracts as the length of a protein is shortened by the denaturation of a collagen protein due to heat generated by a high frequency current, and a delayed reaction, in which collagen synthesis increases as tissue damaged by high frequency heat is regenerated, to deliver energy to all layers of skin, thereby achieving a high frequency irradiation effect.

FIG. 14 is a diagram obtained by comparing results of expression levels of factors related to skin aging when the electrode unit 300 operates with respect to young skin and aged skin in the mono-mono pattern MM, the mono-bi pattern MB, the bi-mono pattern BM, and the bi-bi pattern BB, according to an embodiment of the inventive concept.

This drawing shows a resulting value that is the result of identifying expression levels of MMPs and β-actin, which are factors related to skin aging, by using western blot that indicates a method of detecting specific proteins by using a specific interaction between proteins.

As shown in FIG. 14, it is indicated that the expression of MMP2/MMP3/MMP9 is significantly higher in aged skin than in young skin.

This expression is greatly reduced by the application of electrical (RF) energy in all four patterns. In particular, it is seen that the most significant decrease is observed in the mono-bi pattern MB.

FIG. 20 is a block diagram of a system including a high frequency current output device 1000, according to still another embodiment of the inventive concept. The system includes the skin treatment device 100, the handpiece 200, the electrode unit 300, and an NE pad.

The skin treatment device 100 includes the first high frequency power generation unit 110, the second high frequency power generation unit 120, and the control unit 140.

The electrode unit 300 includes a first electrode group composed of the one or more first electrodes 310-N and a second electrode group composed of the one or more second electrodes 320-N.

As shown in FIG. 20, a plurality of electrodes within the electrode unit 300 may be classified into a first electrode group connected to the first high frequency power generation unit 110 and a second electrode group connected to the second high frequency power generation unit 120.

The one or more first electrodes 310-N belonging to the first electrode group are connected to an electrode of the first polarity of the first high frequency power generation unit 110, and an electrode of the second polarity, which has a polarity opposite to the first polarity of the first high frequency power generation unit 110, is connected to an NE pad. Accordingly, the one or more first electrodes 310-N of the first electrode group receive a monopolar-type current.

Moreover, an electrode 321-N of the first polarity among one or more second electrodes 320-N belonging to the second electrode group is connected to an electrode of the first polarity of the second high frequency power generation unit 120, and an electrode 322-N of the second polarity, which has a polarity opposite to the first polarity, is connected to an electrode of the second polarity of the second high frequency power generation unit 120. Accordingly, the one or more second electrodes 320-N of the second electrode group receive a bipolar-type current.

Furthermore, the control unit 140 turns on or turns off the operation of the first high frequency power generation unit 110 and the second high frequency power generation unit 120 such that a monopolar-type current and/or a bipolar-type current is simultaneously or sequentially supplied to the electrode unit 300.

In the meantime, the electrode unit 300 may be formed as one or more needles and a needle tip surface. The one or more needles may be used to receive the monopolar-type current or the bipolar-type current, and the needle tip surface may be used for an additional electrode.

Besides, the one or more needles in the electrode unit 300 may be used to receive the monopolar-type current, and the needle tip surface may be variously formed to be used to receive a bipolar-type current.

Steps or operations of the method or algorithm described may be implemented directly in hardware, may be implemented with a software module executable by hardware, or may be implemented by a combination thereof. The software module may reside in a random access memory (RAM), a read only memory (ROM), an erasable programmable ROM (EPROM), an electrically erasable programmable ROM (EEPROM), a flash memory, a hard disk, a removable disk, a CD-ROM, or a computer-readable recording medium well known in the art to which the inventive concept pertains.

## Claims

1. A high frequency output device for delivering a high frequency current to skin comprising:
an electrode unit (300) including one or more first electrodes (310-N) and one or more second electrodes (320-N);
a first high frequency power generation unit (110);
a switching circuit (130); and
a control unit (140) configured to control a switching operation of the switching circuit so as to output, based on power generated from the first high frequency power generation unit, a monopolar-type current, in which the same polarity is applied to the first electrodes and the second electrodes, or a bipolar-type current, in which currents of different polarities are applied;
**characterized in that**, as a monopolar-type current is provided, the control unit (140) is configured to control the switching operation of the switching circuit (130) to supply power alternately to the first electrodes (310-N) and
the second electrodes (320-N) with an interval therebetween.

2. The high frequency output device of claim 1, comprising a user interface, wherein the control unit (140) is configured to allow the monopolar-type current and the bipolar-type current to be output in an output setting pattern, which is selected by a user or automatically set,
wherein the monopolar-type current generally increases a temperature of a current supply area, and the bipolar-type current locally increases a temperature of a local area by supplying energy to the local area, and wherein the output setting pattern is set by a combination of high frequency irradiation characteristics as the bipolar-type current and the monopolar-type current are applied simultaneously or sequentially.

3. The high frequency output device for delivering a high frequency current to skin of claim 1, comprising a user interface,
wherein the control unit (140) is configured to allow the pulse duration and energy output intensity of each of the monopolar-type current and the bipolar-type current to be adjusted depending on a current output option selected by a user through the user interface.

4. The high frequency output device for delivering a high frequency current to skin of claim 2, wherein a pattern output in the output setting pattern includes at least one of:
a first pattern in which only the monopolar-type current is output;
a second pattern in which only the bipolar-type current is output;
a third pattern in which the monopolar-type current is output and then the bipolar-type current is output;
a fourth pattern in which the bipolar-type current is output and then the monopolar-type current is output;
a fifth pattern in which the bipolar-type current is output after the monopolar-type current is output, and then the monopolar-type current is output again; and
a sixth pattern in which the monopolar-type current is output after the bipolar-type current is output, and then the bipolar-type current is output again.

5. The high frequency output device for delivering a high frequency current to skin of claim 4, wherein, in a case of the first pattern, an electrode of a first polarity of the first high frequency power generation unit (110) is simultaneously connected to the first electrodes (310-N) and the second electrodes (320-N), an electrode of a second polarity of the first high frequency power generation unit opposite to the first polarity is connected to a neutral electrode (NE) pad, and thus the control unit (140) is configured to control a switching operation of the switching circuit (130) such that a current is applied between the NE pad and the first electrodes and the second electrodes.

6. The high frequency output device for delivering a high frequency current to skin of claim 4, wherein, in a case of the second pattern, an electrode of a first polarity of the first high frequency power generation unit (110) is connected to the first electrodes (310-N), an electrode of a second polarity of the first high frequency power generation unit, which is opposite to the first polarity, is connected to the second electrodes (320-N), and thus the control unit (140) is configured to control a switching operation of the switching circuit (130) such that a current is applied between the first electrodes and the second electrodes.

7. The high frequency output device for delivering a high frequency current to skin of claim 4, wherein, in a case of the third pattern or the fourth pattern, when the monopolar-type current is output, an electrode of a first polarity of the first high frequency power generation unit (110) is connected to the first electrodes (310-N) and the second electrodes (320-N) through controlling a switching operation of the switching circuit (130), an electrode of a second polarity of the first high frequency power generation unit, which is opposite to the first polarity of the first high frequency power generation unit, is connected to an NE pad through controlling a switching operation of the switching circuit, and thus the control unit (140) is configured to allow a current to flow between the NE pad and the first electrodes and the second electrodes, and when the bipolar-type current is output, the electrode of the second polarity of the first high frequency power generation unit is connected to the second electrodes through controlling the switching operation of the switching circuit, and thus the control unit is configured to allow a current to flow between the first electrodes and the second electrodes.

8. The high frequency output device for delivering a high frequency current to skin of claim 4,
wherein, in a case of the third pattern and the fourth pattern, under control of the control unit (140), is configured to measure monopolar-type impedance and bipolar-type impedance of a tissue are measured immediately before and after a first shot, set power is delivered, and power is adjusted through monopolar-type impedance matching and bipolar-type impedance matching at a second shot.

9. The high frequency output device for delivering a high frequency current to skin of claim 1,
wherein the electrode unit (300) is classified into an electrode group for outputting the monopolar-type current and an electrode group for outputting the bipolar-type current, and
wherein the control unit (140) is configured to simultaneously output the monopolar-type current and the bipolar-type current for each group.

10. The high frequency output device for delivering a high frequency current to skin of claim 1,
wherein the electrode unit (300) is formed on a needle tip surface, or a plurality of needles are formed on the first electrodes (310-N) and the second electrodes (320-N), and
wherein the first electrodes and the second electrodes are disposed in a zigzag shape to supply a current to skin, and are connected to different layers insulated from each other to supply a current.

11. The high frequency output device for delivering a high frequency current to skin of claim 10,
wherein, when the electrode unit (300) is formed in a type of a plurality of needles, the control unit (140) is configured to output a pattern in which the bipolar-type current and the monopolar-type current are changed to each other is output, in a process of inserting the needle.

12. The high frequency output device for delivering a high frequency current to skin of claim 1, wherein the electrode unit (300) includes a first electrode group composed of the one or more first electrodes (310-N) and a second electrode group composed of the one or more second electrodes (320-N), a second high frequency power generation unit connected to a separate electrode from the first high frequency power generation unit to supply a current,
the electrode unit (300) further comprises a neutral electrode pad, NE, separate from the first electrode (310-N) and the second electrode (320-N),
wherein the control unit (140) that allows operations of the first high frequency power generation unit (110) and the second high frequency power generation unit (120), which is connected to a separate electrode from the first high frequency power generation unit to supply a current, to be turned on or off such that the monopolar-type current and the bipolar-type current are applied to the electrode unit simultaneously or sequentially,
wherein the first electrode group is connected to the first high frequency power generation unit to form a closed circuit in correspondence with the NE pad for an output of the monopolar-type current, and
wherein the second electrode group is connected to the second high frequency power generation unit (120), and is an electrode for an output of the bipolar-type current.

## Patentansprüche

1. Hochfrequenz-Ausgabevorrichtung zur Abgabe eines Hochfrequenzstroms an Haut, aufweisend:
eine Elektrodeneinheit (300) mit einer oder mehreren ersten Elektroden (310-N) und einer oder mehreren zweiten Elektroden (320-N);
eine erste Hochfrequenz-Stromerzeugungseinheit (110);
ein Schaltkreis (130); und
eine Steuereinheit (140), die dazu ausgebildet ist, einen Schaltvorgang des Schaltkreises so zu steuern, dass auf der Grundlage der von der ersten Hochfrequenz-Stromerzeugungseinheit erzeugten Leistung entweder ein Strom vom monopolaren Typ, bei dem an die ersten Elektroden und die zweiten Elektroden die gleiche Polarität angelegt wird, oder ein Strom vom bipolaren Typ ausgegeben wird, bei dem Ströme unterschiedlicher Polarität angelegt werden;
**dadurch gekennzeichnet, dass** bei Bereitstellung eines Stroms vom monopolaren Typ die Steuereinheit (140) dazu ausgebildet ist, den Schaltvorgang des Schaltkreises (130) so zu steuern, dass die ersten Elektroden (310-N) und die zweiten Elektroden (320-N) abwechselnd mit einem dazwischenliegenden Intervall mit Leistung versorgt werden.

2. Hochfrequenz-Ausgabevorrichtung nach Anspruch 1, die eine Benutzerschnittstelle aufweist,
wobei die Steuereinheit (140) so ausgebildet ist, dass der Strom vom monopolaren Typ und der Strom vom bipolaren Typ in einem Ausgabeeinstellungsmuster ausgegeben werden können, das von einem Benutzer ausgewählt oder automatisch eingestellt wird,
wobei der Strom vom monopolaren Typ im Allgemeinen eine Temperatur eines Stromversorgungsbereichs erhöht, und der Strom vom bipolaren Typ eine Temperatur eines lokalen Bereichs lokal erhöht, indem er diesem lokalen Bereich Energie zuführt, und wobei das Ausgabeeinstellungsmuster durch eine Kombination von Hochfrequenz-Bestrahlungseigenschaften festgelegt wird, wenn der Strom vom bipolaren Typ und der Strom vom monopolaren Typ gleichzeitig oder nacheinander angelegt werden.

3. Hochfrequenz-Ausgabevorrichtung zur Abgabe eines Hochfrequenzstroms an Haut nach Anspruch 1, die eine Benutzerschnittstelle aufweist,
wobei die Steuereinheit (140) so ausgebildet ist, dass die Impulsdauer und die Energieausgabeintensität sowohl des Stroms vom monopolaren Typ als auch des Stroms vom bipolaren Typ in Abhängigkeit von einer vom Benutzer über die Benutzerschnittstelle ausgewählten Stromausgabeoption angepasst werden können.

4. Hochfrequenz-Ausgabevorrichtung zur Abgabe eines Hochfrequenzstroms an Haut nach Anspruch 2, wobei eine Musterausgabe in dem Ausgabeeinstellungsmuster mindestens eines aus Folgendem aufweist:
ein erstes Muster, in dem ausschließlich der Strom vom monopolaren Typ ausgegeben wird;
ein zweites Muster, in dem ausschließlich der Strom vom bipolaren Typ ausgegeben wird;
ein drittes Muster, bei dem zunächst der Strom vom monopolaren Typ und anschließend der Strom vom bipolaren Typ ausgegeben wird;
ein viertes Muster, bei dem zunächst der Strom vom bipolaren Typ und anschließend der Strom vom monopolaren Typ ausgegeben wird;
ein fünftes Muster, bei dem der Strom vom bipolaren Typ nach dem Strom vom monopolaren Typ ausgegeben wird und anschließend der Strom vom monopolaren Typ erneut ausgegeben wird; und
ein sechstes Muster, bei dem der Strom vom monopolaren Typ nach dem Strom vom bipolaren Typ ausgegeben wird und anschließend der Strom vom bipolaren Typ erneut ausgegeben wird.

5. Hochfrequenz-Ausgabevorrichtung zur Abgabe eines Hochfrequenzstroms an Haut nach Anspruch 4, wobei im Fall des ersten Musters eine Elektrode einer ersten Polarität der ersten Hochfrequenz-Stromerzeugungseinheit (110) gleichzeitig mit den ersten Elektroden (310-N) und den zweiten Elektroden (320-N) verbunden ist, eine Elektrode der ersten Hochfrequenz-Stromerzeugungseinheit mit einer zweiten Polarität, die der ersten Polarität entgegengesetzt ist, mit einem Neutralelektroden-(NE)-Pad verbunden ist, und somit die Steuereinheit (140) so ausgebildet ist, dass sie einen Schaltvorgang des Schaltkreises (130) so steuert, dass ein Strom zwischen dem NE-Pad und den ersten Elektroden sowie den zweiten Elektroden angelegt wird.

6. Hochfrequenz-Ausgabevorrichtung zur Abgabe eines Hochfrequenzstroms an Haut nach Anspruch 4, wobei im Fall des zweiten Musters eine Elektrode einer ersten Polarität der ersten Hochfrequenz-Stromerzeugungseinheit (110) mit den ersten Elektroden (310-N) verbunden ist, eine Elektrode der ersten Hochfrequenz-Stromerzeugungseinheit mit einer zweiten Polarität, die der ersten Polarität entgegengesetzt ist, mit den zweiten Elektroden (320-N) verbunden ist, und somit die Steuereinheit (140) so ausgebildet ist, dass sie einen Schaltvorgang des Schaltkreises (130) so steuert, dass ein Strom zwischen den ersten Elektroden und den zweiten Elektroden angelegt wird.

7. Hochfrequenz-Ausgabevorrichtung zur Abgabe eines Hochfrequenzstroms an Haut nach Anspruch 4, wobei im Fall des dritten Musters oder des vierten Musters, wenn der Strom vom monopolaren Typ ausgegeben wird, eine Elektrode einer ersten Polarität der ersten Hochfrequenz-Stromerzeugungseinheit (110) mit den ersten Elektroden (310-N) und den zweiten Elektroden (320-N) durch Steuerung eines Schaltvorgangs des Schaltkreises (130) verbunden ist, eine Elektrode der ersten Hochfrequenz-Stromerzeugungseinheit mit einer zweiten Polarität, die der ersten Polarität der ersten Hochfrequenz-Stromerzeugungseinheit entgegengesetzt ist, mit einem NE-Pad durch Steuerung eines Schaltvorgangs des Schaltkreises verbunden ist, und somit die Steuereinheit (140) so ausgebildet ist, dass sie einem Strom ermöglicht, zwischen den ersten Elektroden und den zweiten Elektroden zu fließen.

8. Hochfrequenz-Ausgabevorrichtung zur Abgabe eines Hochfrequenzstroms an Haut nach Anspruch 4,
wobei im Fall des dritten Musters und des vierten Musters unter der Steuerung der Steuereinheit (140) diese so ausgebildet ist, dass unmittelbar vor und nach einem ersten Impuls die monopolare Impedanz und die bipolare Impedanz eines Gewebes gemessen werden, die eingestellte Leistung abgegeben wird und die Leistung bei einem zweiten Impuls durch monopolare Impedanzanpassung und bipolare Impedanzanpassung angepasst wird.

9. Hochfrequenz-Ausgabevorrichtung zur Abgabe eines Hochfrequenzstroms an Haut nach Anspruch 1,
wobei die Elektrodeneinheit (300) in eine Elektrodengruppe zur Abgabe des Stroms vom monopolaren Typ und eine Elektrodengruppe zur Abgabe des Stroms vom bipolaren Typ unterteilt ist, und
wobei die Steuereinheit (140) so ausgebildet ist, dass sie für jede Gruppe gleichzeitig den Strom vom monopolaren Typ und den Strom vom bipolaren Typ ausgibt.

10. Hochfrequenz-Ausgabevorrichtung zur Abgabe eines Hochfrequenzstroms an Haut nach Anspruch 1,
wobei die Elektrodeneinheit (300) auf einer Nadelspitzenoberfläche ausgebildet ist oder eine Vielzahl von Nadeln an den ersten Elektroden (310-N) und den zweiten Elektroden (320-N) ausgebildet sind, und
wobei die ersten Elektroden und die zweiten Elektroden in einer Zickzackform angeordnet sind, um der Haut Strom zuzuführen, und zur Stromzufuhr mit verschiedenen, voneinander isolierten Schichten verbunden sind.

11. Hochfrequenz-Ausgabevorrichtung zur Abgabe eines Hochfrequenzstroms an Haut nach Anspruch 10,
wobei, wenn die Elektrodeneinheit (300) in Form einer Vielzahl von Nadeln ausgebildet ist, die Steuereinheit (140) so ausgebildet ist, dass sie während des Einführvorgangs der Nadel ein Muster ausgibt, bei dem der Strom vom bipolaren Typ und der Strom vom monopolaren Typ abwechselnd ausgegeben werden.

12. Hochfrequenz-Ausgabevorrichtung zur Abgabe eines Hochfrequenzstroms an Haut nach Anspruch 1, wobei die Elektrodeneinheit (300) eine erste Elektrodengruppe, bestehend aus einer oder mehreren ersten Elektroden (310-N), und eine zweite Elektrodengruppe, bestehend aus einer oder mehreren zweiten Elektroden (320-N), aufweist, wobei eine zweite Hochfrequenz-Stromerzeugungseinheit mit einer von der ersten Hochfrequenz-Stromerzeugungseinheit getrennten Elektrode verbunden ist, um einen Strom zu liefern,
wobei die Elektrodeneinheit (300) ferner ein Neutralelektrodenpad, NE, aufweist, das von der ersten Elektrode (310-N) und der zweiten Elektrode (320-N) getrennt ist, wobei die Steuereinheit (140), die den Betrieb der ersten Hochfrequenz-Stromerzeugungseinheit (110) und der zweiten Hochfrequenz-Stromerzeugungseinheit (120) ermöglicht, die mit einer von der ersten Hochfrequenz-Stromerzeugungseinheit getrennten Elektrode verbunden ist, um einen Strom zu liefern, so ein- und ausgeschaltet werden kann, dass der Strom vom monopolaren Typ und der Strom vom bipolaren Typ gleichzeitig oder nacheinander an die Elektrodeneinheit angelegt werden, wobei die erste Elektrodengruppe mit der ersten Hochfrequenz-Stromerzeugungseinheit verbunden ist, um entsprechend dem NE-Pad einen geschlossenen Stromkreis zur Ausgabe des Stroms vom monopolaren Typ zu bilden, und
wobei die zweite Elektrodengruppe mit der zweiten Hochfrequenz-Stromerzeugungseinheit (120) verbunden ist und eine Elektrode zur Ausgabe eines bipolaren Stroms darstellt.

## Revendications

1. Dispositif de sortie haute fréquence destiné à administrer un courant à haute fréquence à de la peau, comprenant :
une unité d'électrodes (300) comprenant une ou plusieurs premières électrodes (310-N) et une ou plusieurs secondes électrodes (320-N) ;
une première unité de production de puissance haute fréquence (110) ;
un circuit de commutation (130) ; et
une unité de commande (140) configurée pour commander une opération de commutation du circuit de commutation de façon à délivrer, sur la base d'une puissance produite par la première unité de production de puissance haute fréquence, un courant de type monopolaire, dans lequel la même polarité est appliquée aux premières électrodes et aux secondes électrodes, ou un courant de type bipolaire, dans lequel des courants de polarités différentes sont appliqués ;
**caractérisé en ce que**, lorsqu'un courant de type monopolaire est fourni, l'unité de commande (149) est configurée pour commander l'opération de commutation du circuit de commutation (130) pour appliquer de la puissance de manière alternée aux premières électrodes (310-N) et aux secondes électrodes (320-N) avec un intervalle de séparation.

2. Dispositif de sortie haute fréquence selon la revendication 1, comprenant une interface utilisateur, dans lequel l'unité de commande (140) est configurée pour permettre une sortie selon un motif de définition de sortie du courant de type monopolaire et du courant de type bipolaire, qui est sélectionné par un utilisateur ou qui est défini automatiquement,
dans lequel le courant de type monopolaire augmente globalement une température d'une zone d'alimentation en courant, et le courant de type bipolaire augmente localement une température d'une zone locale en délivrant de l'énergie à la zone locale, et dans lequel le motif de définition de sortie est défini par une combinaison de caractéristiques de rayonnement haute fréquence lorsque le courant de type bipolaire et le courant de type monopolaire sont appliqués simultanément ou successivement.

3. Dispositif de sortie haute fréquence destiné à administrer un courant à haute fréquence à de la peau selon la revendication 1, comprenant une interface utilisateur,
dans lequel l'unité de commande (140) est configurée pour permettre un réglage de la durée d'impulsion et de l'intensité de sortie d'énergie de chacun du courant de type monopolaire et du courant de type bipolaire en fonction d'une option de sortie de courant sélectionnée par un utilisateur par l'intermédiaire de l'interface utilisateur.

4. Dispositif de sortie haute fréquence destiné à administrer un courant à haute fréquence à de la peau selon la revendication 2, dans lequel un motif délivré selon le motif de définition de sortie comprend au moins l'un :
d'un premier motif dans lequel n'est délivré que le courant de type monopolaire ;
d'un deuxième motif dans lequel n'est délivré que le courant de type bipolaire ;
d'un troisième motif dans lequel est délivré le courant de type monopolaire puis le courant de type bipolaire ;
d'un quatrième motif dans lequel est délivré le courant de type bipolaire puis le courant de type monopolaire ;
d'un cinquième motif dans lequel le courant de type bipolaire est délivré après la sortie du courant de type monopolaire, puis le courant de type monopolaire est délivré à nouveau ; et
d'un sixième motif dans lequel le courant de type monopolaire est délivré après la sortie du courant de type bipolaire, puis le courant de type bipolaire est délivré à nouveau.

5. Dispositif de sortie haute fréquence destiné à administrer un courant à haute fréquence à de la peau selon la revendication 4, dans lequel, dans un cas du premier motif, une électrode d'une première polarité de la première unité de production de puissance haute fréquence (110) est simultanément connectée aux premières électrodes (310-N) et aux secondes électrodes (320-N), une électrode d'une seconde polarité de la première unité de production de puissance haute fréquence contraire à la première polarité est connectée à une pastille d'électrode neutre (NE), et ainsi l'unité de commande (140) est configurée pour commander une opération de commutation du circuit de commutation (130) de sorte qu'un courant est appliqué entre la pastille de NE et les premières électrodes et les secondes électrodes.

6. Dispositif de sortie haute fréquence destiné à administrer un courant à haute fréquence à de la peau selon la revendication 4, dans lequel, dans un cas du deuxième motif, une électrode d'une première polarité de la première unité de production de puissance haute fréquence (110) est connectée aux premières électrodes (310-N), une électrode d'une seconde polarité de la première unité de production de puissance haute fréquence, qui est contraire à la première polarité, est connectée aux secondes électrodes (320-N), et ainsi l'unité de commande (140) est configurée pour commander une opération de commutation du circuit de commutation (130) de sorte qu'un courant est appliqué entre les premières électrodes et les secondes électrodes.

7. Dispositif de sortie haute fréquence destiné à administrer un courant à haute fréquence à de la peau selon la revendication 4, dans lequel, dans un cas du troisième motif ou du quatrième motif, lorsque le courant de type monopolaire est délivré, une électrode d'une première polarité de la première unité de production de puissance haute fréquence (110) est connectée aux premières électrodes (310-N) et aux secondes électrodes (320-N) par l'intermédiaire d'une commande d'une opération de commutation du circuit de commutation (130), une électrode d'une seconde polarité de la première unité de production de puissance haute fréquence, qui est contraire à la première polarité de la première unité de production de puissance haute fréquence, est connectée à une pastille de NE par l'intermédiaire d'une commande d'une opération de commutation du circuit de commutation, et ainsi l'unité de commande (140) est configurée pour permettre la circulation d'un courant entre la pastille de NE et les premières électrodes et les secondes électrodes, et lorsque le courant de type bipolaire est délivré, l'électrode de la seconde polarité de la première unité de production de puissance haute fréquence est connectée aux secondes électrodes par l'intermédiaire d'une commande de l'opération de commutation du circuit de commutation, et ainsi l'unité de commande est configurée pour permettre la circulation d'un courant entre les premières électrodes et les secondes électrodes.

8. Dispositif de sortie haute fréquence destiné à administrer un courant à haute fréquence à de la peau selon la revendication 4,
dans lequel, dans un cas de la configuration du troisième motif et du quatrième motif, sous la commande de l'unité de commande (140), pour mesurer une impédance de type monopolaire et une impédance de type bipolaire d'un tissu, ces dernières sont mesurées immédiatement avant et après une première impulsion, une puissance définie est délivrée, et la puissance est réglée par l'intermédiaire d'une adaptation d'impédance de type monopolaire et d'une adaptation d'impédance de type bipolaire lors d'une seconde impulsion.

9. Dispositif de sortie haute fréquence destiné à administrer un courant à haute fréquence à de la peau selon la revendication 1,
dans lequel l'unité d'électrodes (300) est classifiée en un groupe d'électrodes destinées à délivrer le courant de type monopolaire et un groupe d'électrodes destinées à délivrer le courant de type bipolaire, et
dans lequel l'unité de commande (140) est configurée pour délivrer simultanément le courant de type monopolaire et le courant de type bipolaire de chaque groupe.

10. Dispositif de sortie haute fréquence destiné à administrer un courant à haute fréquence à de la peau selon la revendication 1,
dans lequel l'unité d'électrodes (300) est formée sur une surface de pointe d'aiguille, ou les aiguilles d'une pluralité d'aiguilles sont formées sur les premières électrodes (310-N) et sur les secondes électrodes (320-N), et
dans lequel les premières électrodes et les secondes électrodes sont disposées en une forme de zigzag pour délivrer un courant à de la peau, et sont connectées à différentes couches isolées les unes des autres pour délivrer un courant.

11. Dispositif de sortie haute fréquence destiné à administrer un courant à haute fréquence à de la peau selon la revendication 10,
dans lequel, lorsque l'unité d'électrodes (300) est formée selon un type d'une pluralité d'aiguilles, l'unité de commande (140) est configurée pour délivrer un motif selon lequel le courant de type bipolaire et le courant de type monopolaire sont interchangés, lors d'un processus d'insertion de l'aiguille.

12. Dispositif de sortie haute fréquence destiné à administrer un courant à haute fréquence à de la peau selon la revendication 1, dans lequel l'unité d'électrodes (300) comprend un premier groupe d'électrodes composé de la ou des premières électrodes (310-N) et un second groupe d'électrodes composé de la ou des secondes électrodes (320-N), une seconde unité de production de puissance haute fréquence connectée à une électrode distincte de la première unité de production de puissance haute fréquence pour délivrer un courant,
l'unité d'électrodes (300) comprend en outre une pastille d'électrode neutre, dite NE, distincte de la première électrode (310-N) et de la seconde électrode (320-N),
dans lequel l'unité de commande (140) qui autorise l'activation ou la désactivation de fonctionnements de la première unité de production de puissance haute fréquence (110) et de la seconde unité de production de puissance haute fréquence (120), qui est connectée à une électrode distincte de la première unité de production de puissance haute fréquence pour délivrer un courant, de sorte que le courant de type monopolaire et le courant de type bipolaire sont appliqués simultanément ou successivement à l'unité d'électrodes,
dans lequel le premier groupe d'électrodes est connecté à la première unité de production de puissance haute fréquence pour former un circuit fermé en correspondance avec la pastille de NE à des fins de sortie du courant de type monopolaire, et dans lequel le second groupe d'électrodes est connecté à la seconde unité de production de puissance haute fréquence (120), et est une électrode destinée à une sortie du courant de type bipolaire.
